# EUROPEAN PATENT SPECIFICATION

(11) **EP 3 039 128 B1**
(45) Date of publication and mention of the grant of the patent: **19.12.2018**
(21) Application number: 14783549.0
(22) Date of filing: 28.08.2014
(51) Int. Cl.: C12N 7/00, C12Q 1/70, G01N 33/569

(54) **METHOD FOR PROPAGATING A VIRUS**
VERFAHREN ZUR AUSBREITUNG EINES VIRUS
PROCÉDÉ DE PROPAGATION D'UN VIRUS

(30) Priority: 30.08.2013 NO 20131163; 16.12.2013 DK 201300697; 21.02.2014 SE 1450212
(43) Date of publication of application: 06.07.2016
(73) Proprietor: Pharmaq AS, 7863 Overhalla (NO)
(72) Inventor: FINSTAD, Øystein W., 1482 Nittedal (NO); DAHLE, Maria K., N-1404 Siggerud (NO); RIMSTAD, Espen, N-1361 Østerås (NO)
(74) Representative: Mannion, Sally Kim
(86) International application number: PCT/EP2014/068293
(87) International publication number: WO 2015/028565

(56) References cited:
- WO-A1-2005/121325
- WO-A1-2011/041790
- AASE B. MIKALSEN ET AL: "Atlantic Salmon Reovirus Infection Causes a CD8 T Cell Myocarditis in Atlantic Salmon (Salmo salar L.)", PLOS ONE, vol. 7, no. 6, January 2012 (2012-01), page e37269, XP055059355, ISSN: 1932-6203, DOI: 10.1371/journal.pone.0037269 cited in the application
- Å. H. GARSETH ET AL: "Piscine reovirus (PRV) in wild Atlantic salmon, Salmo salar L., and sea-trout, Salmo trutta L., in Norway", JOURNAL OF FISH DISEASES, vol. 36, no. 5, 20 May 2013 (2013-05-20), pages 483-493, XP055118434, ISSN: 0140-7775, DOI: 10.1111/j.1365-2761.2012.01450.x cited in the application
- Ã YSTEIN WESSEL FINSTAD ET AL: "Immunohistochemical detection of piscine reovirus (PRV) in hearts of Atlantic salmon coincide with the course of heart and skeletal muscle inflammation (HSMI)", VETERINARY RESEARCH, BIOMED CENTRAL LTD, LONDON, UK, vol. 43, no. 1, 9 April 2012 (2012-04-09), page 27, XP021094756, ISSN: 1297-9716, DOI: 10.1186/1297-9716-43-27 cited in the application
- M LØVOLL ET AL: "Quantification of piscine reovirus (PRV) at different stages of Atlantic salmon Salmo salar production", DISEASES OF AQUATIC ORGANISMS, vol. 99, no. 1, 15 May 2012 (2012-05-15), pages 7-12, XP055118450, ISSN: 0177-5103, DOI: 10.3354/dao02451 cited in the application
- MAX L. NIBERT ET AL: "Bioinformatics of Recent Aqua- and Orthoreovirus Isolates from Fish: Evolutionary Gain or Loss of FAST and Fiber Proteins and Taxonomic Implications", PLOS ONE, vol. 8, no. 7, 4 July 2013 (2013-07-04), page e68607, XP055157572, DOI: 10.1371/journal.pone.0068607 cited in the application
- ØYSTEIN WESSEL FINSTAD ET AL: "Piscine orthoreovirus (PRV) infects Atlantic salmon erythrocytes", VETERINARY RESEARCH, vol. 45, 3 April 2014 (2014-04-03), page 35, XP055118198,
- ICTV: "Reoviridae", NCBI , Retrieved from the Internet: URL:https://www.ncbi.nlm.nih.gov/Taxonomy/ Browser/wwwtax.cgi?mode=Undef&id=10880&lvl =3&keep=1&srchmode=1&unlock [retrieved on 2017-07-20]

### SEQUENCE LISTING

<110> Veterinærinstituttet
<120> Method for propagating a virus
<130> PG20144PC00
<150> NO 20131163
   <151> 2013-08-30
<150> SE 1450212-4
   <151> 2014-02-21
<150> PA 2013 00697
   <151> 2013-12-16
<160> 14
<170> PatentIn version 3.5
<210> 1
   <211> 20
   <212> DNA
   <213> artificial
<220>
   <223> primer
<400> 1
   tgcgtcctgc gtatggcacc 20
<210> 2
   <211> 21
   <212> DNA
   <213> artificial
<220>
   <223> primer
<400> 2
   ggctggcatg cccgaatagc a 21
<210> 3
   <211> 16
   <212> DNA
   <213> artificial
<220>
   <223> probe
<400> 3
   atcacaacgc ctacct 16
<210> 4
   <211> 26
   <212> DNA
   <213> artificial
<220>
   <223> primer
<400> 4
   actgaaacgc tacttcaaga agttga 26
<210> 5
   <211> 25
   <212> DNA
   <213> artificial
<220>
   <223> primer
<400> 5
   gcagatgacg ttttgtctct ttcct 25
<210> 6
   <211> 24
   <212> DNA
   <213> artificial
<220>
   <223> probe
<400> 6
   ttgatgggct ggatgacttt agga 24
<210> 7
   <211> 24
   <212> DNA
   <213> artificial
<220>
   <223> primer
<400> 7
   gatgctgcac ctcaagtcct atta 24
<210> 8
   <211> 21
   <212> DNA
   <213> artificial
<220>
   <223> primer
<400> 8
   caccaggtag cggatcacca t 21
<210> 9
   <211> 30
   <212> DNA
   <213> artificial
<220>
   <223> probe
<400> 9
   ctgatcagcc aaacgttgac tggatatcct 30
<210> 10
   <211> 20
   <212> DNA
   <213> artificial
<220>
   <223> primer
<400> 10
   acgccttgaa gagctggata 20
<210> 11
   <211> 20
   <212> DNA
   <213> artificial
<220>
   <223> primer
<400> 11
   ctggctggac ttgtgtcctc 20
<210> 12
   <211> 26
   <212> DNA
   <213> artificial
<220>
   <223> probe
<400> 12
   cctgaaacca ggagtactga tcggga 26
<210> 13
   <211> 20
   <212> DNA
   <213> artificial
<220>
   <223> primer
<400> 13
   tgcccctcca ggatgtctac 20
<210> 14
   <211> 18
   <212> DNA
   <213> artificial
<220>
   <223> primer
<400> 14
   cacggcccac aggtactg 18

## Description

### TECHNICAL FIELD

The present disclosure relates to the field of aquaculture, fish farming and infectious diseases appearing in dense populations of farmed fish. Particularly, it relates to a method for propagating a virus causing disease in *Salmonidae.*

### BACKGROUND

Aquaculture is the fastest growing food producing sector in the world, and fish farming will be a key contributor to meet the growing demand for animal proteins. However, intensive production, including rearing fish in dense populations, has led to the emergence of several new infectious diseases.
Heart and skeletal muscle inflammation (HSMI), first detected in 1999, is an increasingly important disease in farmed Atlantic salmon (*Salmosalar L.).* Salmon is a *Salmonidae,* a family of ray-finned fish, which is the only living family currently placed in the order Salmoniformes. These may also be referred to as salmonids. *Salmonidae* includes salmon, trout, chars, freshwater whitefishes and graylings. HSMI is discussed by (1) HSMI usually occurs 5-9 months after transfer of the fish to seawater, and is characterized by epi-, endo- and myocarditis, myocardial necrosis, myositis and necrosis of the red skeletal muscle. The cumulative mortality may reach 20%, but the morbidity is higher as most fish in an affected sea cage show histopathological lesions in the heart. Pathological changes in the heart are also seen in other diseases in Atlantic salmon, including pancreas disease (PD) and cardiomyopathy syndrome (CMS). (2) HSMI was recently found to be associated with a novel reovirus, piscine orthoreovirus also named piscine reovirus (PRV). The virus genome was identified using high throughput sequencing (6) disclose the PRV genome. Reoviruses belong to the family *Reoviridae* and are commonly known to infect the gastrointestinal system and respiratory tract. Piscine reovirus belongs to an as yet classified genus in the subfamily *Spinareovirinae* (3).

Piscine orthoreovirus (PRV) has a segmented double stranded RNA genome, belongs to the Reoviridae family and is the only known fish virus related to the Orthoreovirus genus. Other known fish reoviruses are grouped in the genus Aquareovirus, but phylogenetic analysis indicate that PRV branches off the common root of the genera Orthoreovirus and Aquareovirus. PRV has 10 genomic segments, as do the orthoreoviruses, but the overall amino acid identity between the homologous proteins is very low, particularly for the surface-exposed and non-structural proteins. However, several amino acid motifs central to protein function are conserved for orthoreoviruses and PRV. Unlike most orthoreoviruses, but similar to the mammalian orthoreoviruses (MRV), PRV is non-fusogenic (4,5). PRV is ubiquitously distributed in Atlantic salmon, farmed and wild, and high loads of virus in the heart are consistent findings in HSMI outbreaks. Ubiquitous presence of PRV in farmed Atlantic salmon has been found in Norway, Canada and Chile, and the virus has also been detected in wild Atlantic salmon and brown trout (*Salmo trutta L*.) in Norway, and wild Chum salmon (*Oncorhynchus keta*), Rainbow trout (*O*. *mykiss*) and Cutthrout trout (*O*. *clarkii*) in Canada The mechanism by which PRV infection causes disease remains largely unknown. (6,7)

Finstad *et al.* (8) showed the presence of PRV in cardiomyocytes and blood cells by immunohistochemistry (antibodies against the PRV surface protein sigma1). The presence in blood cells appeared to precede viral detection in cardiomyocytes.

Piscine orthoreovirus (PRV) is thus the causative agent of Heart and skeletal muscle inflammation (HSMI), HSMI is characterized by epi-,endo- and myocarditis with infiltration of mononuclear CD8 positive cells, as well as myositis in red skeletal muscle (9).

PRV encodes a homologous protein of the outer-fiber protein (σ1/σC) that is present in most members of genus Orthoreovirus. In MRV the σ1 protein serve as the viral hemagglutinin and cell attachment protein, and amino acid residues central for this binding are partly conserved in the PRV σ1 protein (4).

Cultivation of PRV in commonly used fish cell lines has not been successful so far. The load of PRV, as measured by RT-qPCR, correlates with disease development in both naturally and experimentally infected salmon.

HSMI is mainly observed during the seawater grow out phase of the fish with morbidity close to 100% in affected cages, while cumulative mortality varies from negligible to 20%. Typical gross pathologic changes in affected fish include signs of circulatory disturbance, with pale heart, ascites, yellow liver, swollen spleen and petechiae in perivascular fat. The diagnosis of HSMI is currently based on histopathological findings of the heart and red skeletal muscle. The viral load in the heart, as measured by RT-qPCR, and the presence of viral proteins in cardiomyocytes demonstrated by immunohistochemistry, both correlate with the development of histological heart lesions, and indicate that PRV replicates in heart tissue. Although the heart lesions has been the main focus in HSMI diagnostics, PRV has been detected in the spleen and head kidney at higher viral load than that of the heart, and inoculates originating from heart, liver, kidney/spleen and blood plasma from diseased fish have been used successfully to reproduce disease (1).

In PRV challenge experiments in salmon there is a relatively long incubation period, i.e. 6 weeks post challenge (wpc) from injection until the first signs of myocarditis are observed, and another 4 weeks before pathological changes occur in cohabitant fish. The inflammatory changes appear to initiate in the outer part of the ventricle, extending from the epicardium and into the compact layer particularly along small vascular structures. In addition, a mild endocarditis is observed in these early phases. In contrast to the inner spongy layer, the outer compact part of the ventricle is highly vascularized. Interestingly, using immunohistochemistry on heart sections from experimentally PRV challenged fish a distinct pattern of viral presence in blood cells prior to cardiac lesions was detected The PRV positive cells detected included both erythrocytes and leukocyte-like cells, but the material available was not suitable for further characterization of these cells. (8)

Previous studies of field material from four separate HSMI outbreaks consistently detected virus like particles (VLP) by electron microscopy (EM) in erythrocytes, and similar VLP were also described in macrophages in the head-kidney (10). VLP of similar morphology have been detected in erythrocytes of many species of salmonid fish and these findings have collectively been called Erythrocytic inclusions body syndrome (EIBS) without a specific etiological notification (11). The EIBS-related VLP inclusions have not been further characterized and it is not documented whether they represent one or several viral species. Accordingly, the presence of PRV viremia has not been verified.

In WO2011041790 there is provided piscine reovirus diagnostic compositions, however alternative sources for retrieving the virus are presented and no proof of a vaccinating effect of the compositions are shown therein.
WO2005121325 describes an isolated Heart and Skeleton Muscle Inflammation Virus (HSMIV) that causes Heart and Skeleton Muscle Inflammation (HSMI) in fish.

Accordingly, propagation of PRV has proven very difficult, and this has been a strong limitation for vaccine development against the virus, and for production of viral antigens. Furthermore, a test system for viral infectivity is also sought after, which would be of great value for testing of diagnostics, disinfectants etc.

Accordingly, there is a need for identifying a way of propagating and obtaining PRV and other orthoreoviruses to be able to overcome or mitigate the problems identified in the art. There is a further need in the art to identify new ways of retrieving larger amounts of orthoreovirus , such as PRV, to be able to produce vaccine products e.g. of inactivated forms of the virus which may be used in the treatment and/or prevention of disease in *Salmonidae,* such as salmon.

### SUMMARY

The invention is as defined in the claims.

The present disclosure has overcome, or at least mitigated, the problems in the prior art by providing an *ex vivo* method for propagating and isolating a piscine reovirus (PRV) using nucleated piscine erythrocytes (i.e. piscine erythrocytes having a cell nucleus). The method comprises *ex vivo* propagating a piscine reovirus (PRV) in nucleated piscine erythrocytes and thereafter isolating the propagated PRV. PRV is a reovirus which belongs to a replicating lineage occupying the ecological niche of nucleated piscine erythrocytes, i.e. reovirus that *in vivo* infects and replicates in nucleated piscine erythrocytes. The nucleated piscine erythrocytes may for example be erythrocytes from a *Salmonidae,* such as salmon, trout, rainbow trout, chars, freshwater whitefishes and graylings, in particular salmon. Said *ex vivo* method for propagating and isolating PRV may comprise the steps of a) co-cultivation of nucleated piscine erythrocytes (such as *Salmonidae* erythrocytes, e.g. erythrocytes from salmon) substantially free from reovirus with nucleated piscine erythrocytes (such as *Salmonidae* erythrocytes, e.g. erythrocytes from salmon) infected with PRV and/or any other material containing a source of PRV, to obtain a mixed culture; b) incubating the mixed culture obtained in step a), c) removing erythrocytes infected with PRV from said mixed culture of step b), d) optionally repeating the procedure of steps a) to c), and e) isolating PRV from the erythrocytes of step c). As mentioned elsewhere herein, the PRV replicates in nucleated piscine erythrocytes, such as *Salmonidae* erythrocytes, such as erythrocytes from salmon.
In such a method, the incubation of step b) may be performed for about 4 days or more, such as about 4-45 day, e.g. about 14 days or more. Further, the procedure of steps a) to d) may be repeated at least 2 times, such as about 2-10 times, but is not limited thereto.
A *Salmonidae* as mentioned herein may be a salmon, trout, rainbow trout, chars, freshwater whitefishes and graylings, in particular a salmon.
Also disclosed herein is an *ex vivo* method of preparing a vaccine composition comprising a PRV, said method comprising *ex vivo* propagating a PRV in nucleated piscine erythrocytes, and thereafter isolating the propagated PRV followed by inactivating the isolated PRV and thereafter preparing a vaccine composition containing said inactivated PRV. The present document is also directed to a vaccine composition prepared by the method for propagating and isolating a PRV as disclosed herein.

Such an *ex vivo* method of preparing a vaccine composition comprising a PRV may comprise the steps of a) co-cultivation of nucleated piscine erythrocytes, such as *Salmonidae* erythrocytes, such as erythrocytes from salmon, substantially free from reovirus, with nucleated piscine erythrocytes, such as *Salmonidae* erythrocytes, such as erythrocytes from salmon, infected with a PRV and/or any other material containing a source of PRV to obtain a mixed culture, b) incubating the mixed culture obtained in step a), c) removing erythrocytes infected with PRV from said mixed culture of step b), d) optionally repeating the procedure of steps a) to c), and e) isolating PRV from the erythrocytes of step c), followed by inactivating the PRV obtained in step e) and thereafter preparing a composition containing said inactivated PRV. As mentioned elsewhere herein, the PRV replicates in nucleated piscine erythrocytes, such as *Salmonidae* erythrocytes, such as erythrocytes from salmon. The PRV vaccine composition may be used for the treatment and/or prevention of Heart and Skeletal Muscle Inflammation (HSMI). There is further provided herein an *ex vivo* method for diagnosing Heart and Skeletal Muscle Inflammation (HSMI) or the presence of PRV virus in fish of a species of *Salmonidae,* said method comprising:
a) isolating erythrocytes from a biological blood sample, b) detecting the presence of piscine reovirus (PRV) in said erythrocytes obtained from step a). Such detection may be performed by RT-qPCR. Detecting the presence of piscine reovirus in erythrocytes is an indication of, or the possible development of, Heart and Skeletal Muscle Inflammation (HSMI).

The present document also discloses the *ex vivo* use of nucleated piscine erythrocytes for propagating piscine reovirus (PRV). The nucleated piscine erythrocytes may be *Salmonidae* erythrocytes, such as salmon erythrocytes or erythrocytes from trout, chars, freshwater whitefishes or graylings.

### BRIEF DESCRIPTION OF THE DRAWINGS

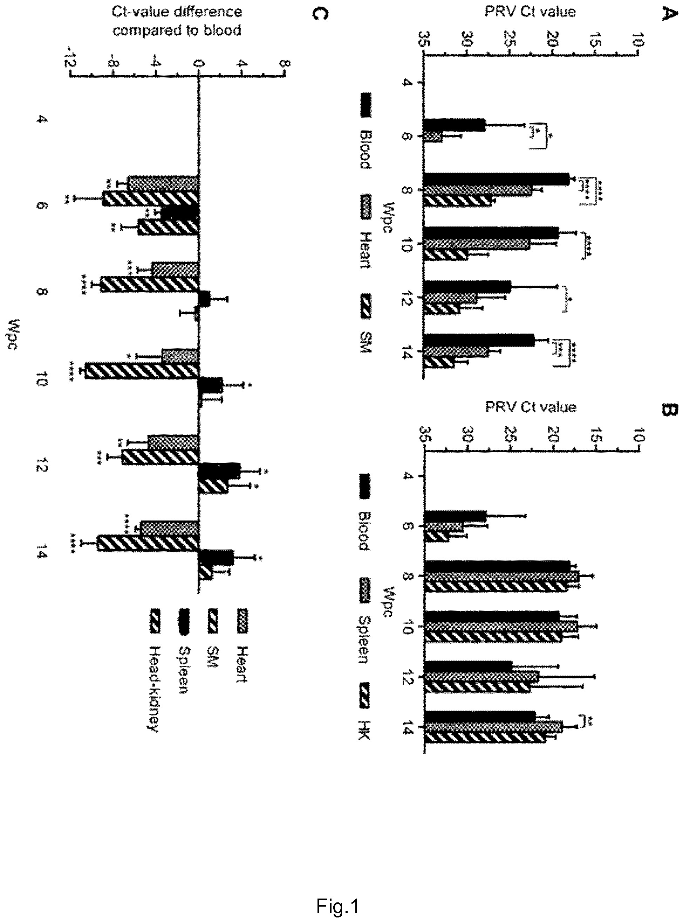

Figure 1. Detection of PRV by RT-qPCR in challenge experiment 1. (A) Mean Ct-values and SD in blood compared to the heart and skeletal muscle (SM). (B) Mean Ct-values and SD in blood compared to the spleen and head kidney (HK). (C) Paired analysis of the PRV Ct-value detected in blood compared to each tissue sampled from the same fish (heart, skeletal muscle, spleen, head-kidney). The results are presented as the mean Ct-value difference at each time point. The data was analyzed using paired T-test. * <0.05; ** <0.01; <0.001***; 0.0001****.

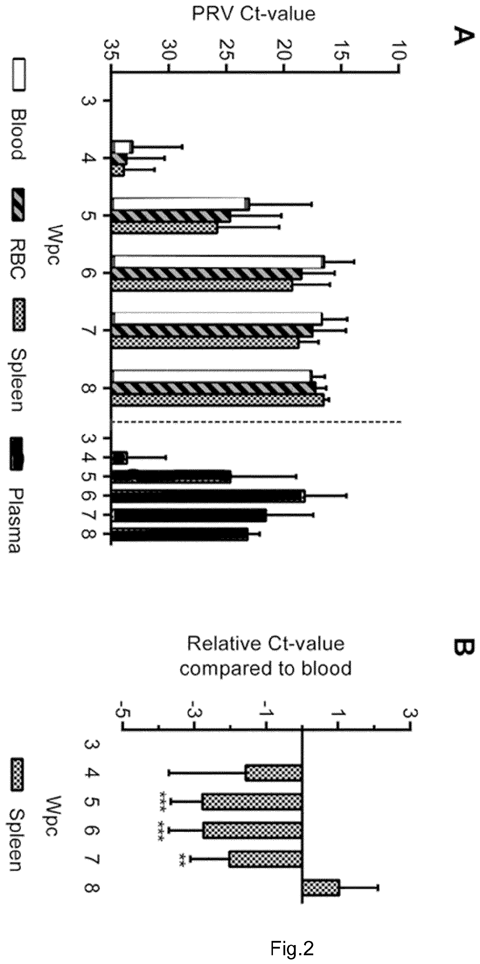

Figure 2. (A) Detection of PRV by RT-qPCR in challenge experiment 2 presented by the mean Ct-values and SD in blood, RBC and spleen. The PCR results from the plasma are shown separately on the right as the RNA input from the cell free plasma is not directly comparable to the other sample material analyzed. (B) Paired analysis of the PRV Ct-value detected in blood compared to spleen from the same fish, presented mean Ct-value difference at each time point. The data was analyzed using paired T-test. * <0.05; ** <0.01; <0.001***.

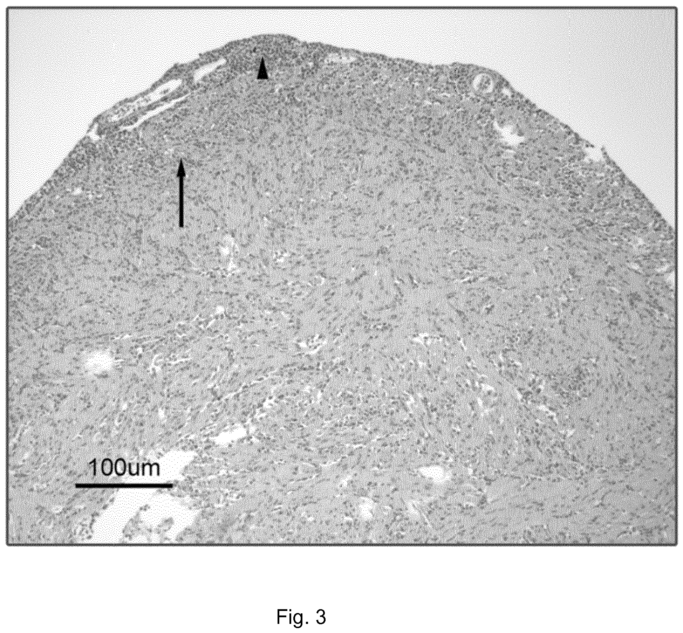

Figure 3. Histopathological changes in the ventricle of the heart at 7 Wpc, including epicarditis (arrowhead) and inflammation of the compactum layer (arrow), consistent with an HSMI diagnosis.

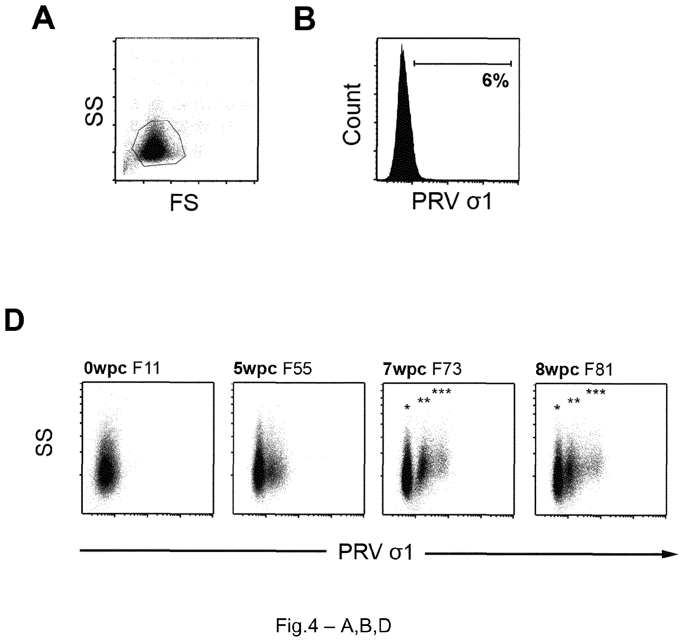

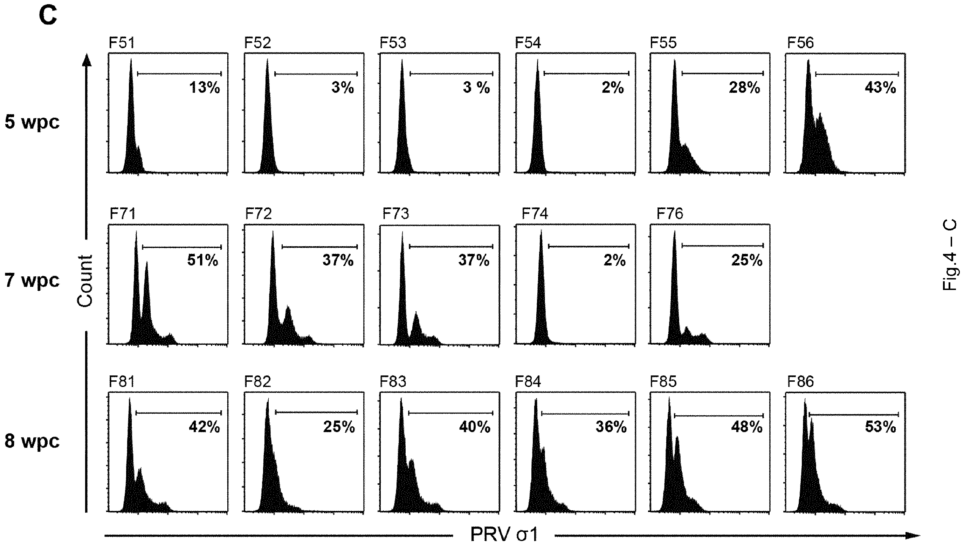

Figure 4. Detection of PRV protein in isolated RBC from Challenge 2 by flow cytometry. (A) Density plot showing the gating strategy for intracellular staining. FS, forward scatter; SS, side scatter. (B) Negative control from 0 wpc for intracellular staining representing the background fluorescence. (C) Flow cytometry result from the intracellular staining at 5, 7 and 8 wpc. 50 000 cells were counted for each sample. Individual F75 was excluded due to technical difficulties. (D) Plots of side scatter (SS) and fluorescence intensity (PRV o1) from 0, 5, 7 and 8 wpc. PRV negative (*), low positive (**) and high positive (***) population are indicated at 7 and 8 wpc.

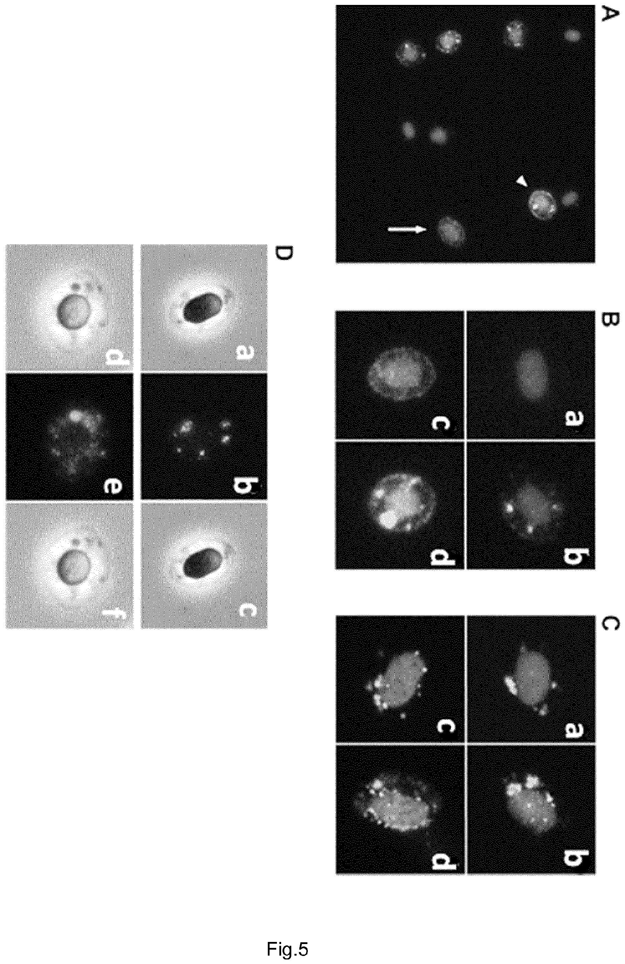

Figure 5. Fluorescent labeling of the PRV o'1-protein in erythrocytes (green). Cell nucleus stained with propidium iodide (red). (A) Positive RBC detected by immunofluorescence (IF) microscopy. Labeled cells varied in morphology from elongated mature RBC (arrow) to more spherical cells resembling immature erythrocytes (arrowhead). (B) IF microscopy of a negative cell (a) and different staining pattern in the cytoplasm including a few smaller inclusions (b), scattered granular staining (c) and large cytoplasmic inclusions (d). (C) Confocalmicroscopy images showing viral inclusions in the perinuclear region (a,b,c) and a more scattered staining pattern (d). (D) Cytoplasmic inclusion detected by phase contrast (a,d), by IF microscopy (b,e) and co-localized by image overlay (c,f).

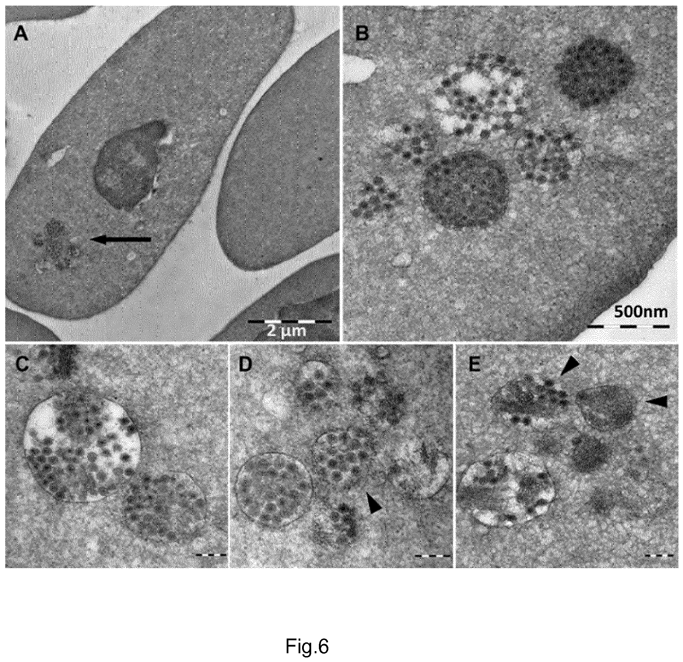

Figure 6. Transmission electron micrographs. A) Erythrocyte with viral inclusions (arrow). B) Inclusions containing reovirus-like particles. C) Inclusions with intact membrane-like structure. D) Inclusions with partly intact or absence of membrane-like structure (arrowhead). E) Inclusions with a mixture of viral particles and lamellar structures (arrowhead).

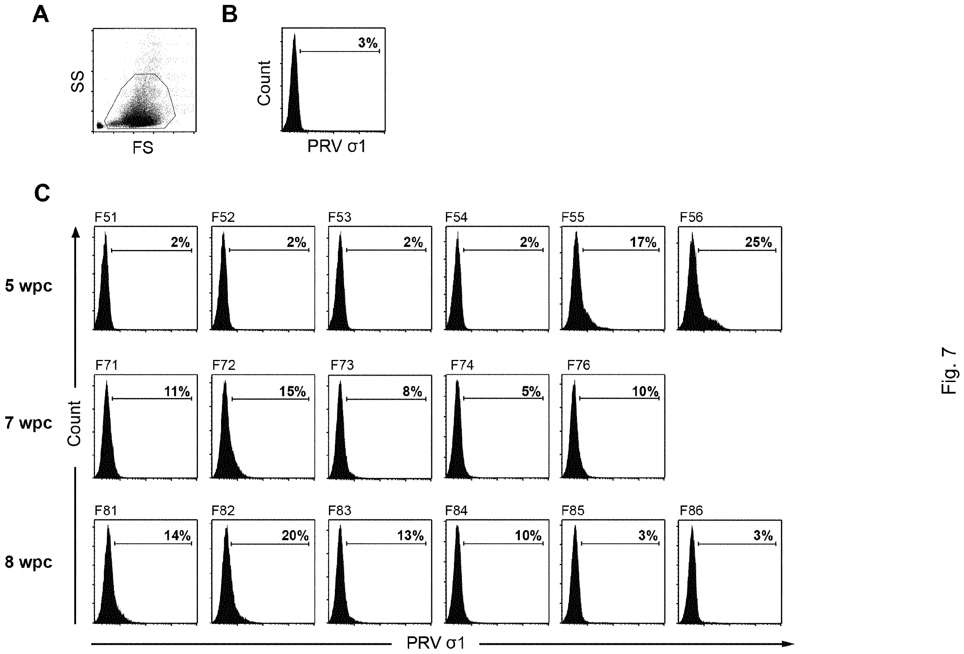

Figure 7. Surface detection of PRV protein in isolated RBC from Challenge 2 by flow cytometry. (A) Density plot showing the gating strategy for surface staining. FS,forward scatter; SS, side scatter. (B) Negative control from 0 wpc for surface staining representing the background fluorescence. (C) Flow cytometry result from the surface staining at 5, 7 and 8 wpc. 50 000 cells were counted for each sample. Individual F75 was excluded due to technical difficulties.

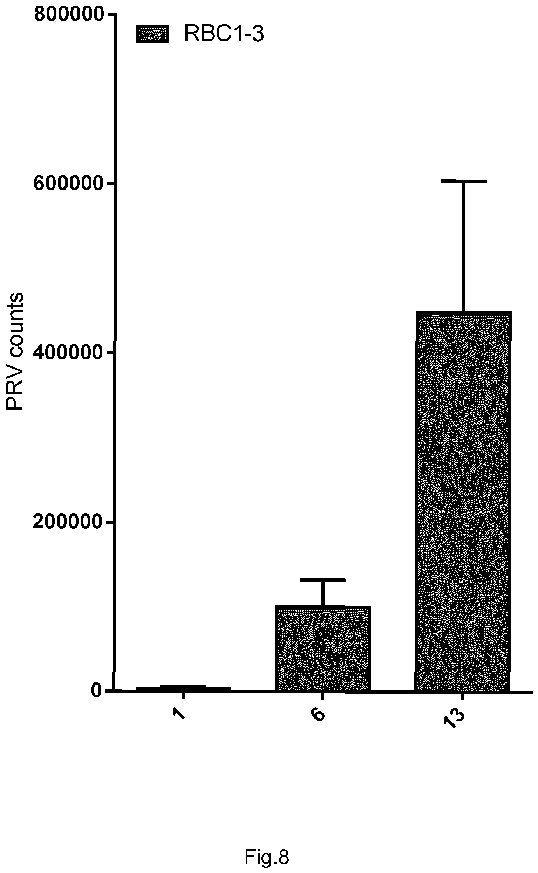

Figure.8 shows the mean increase in viral counts (RT-qPCR absolute quantification) in naïve erythrocytes 6 and 13 days after introduction of virus infected erythrocytes from a fish infected with PRV.

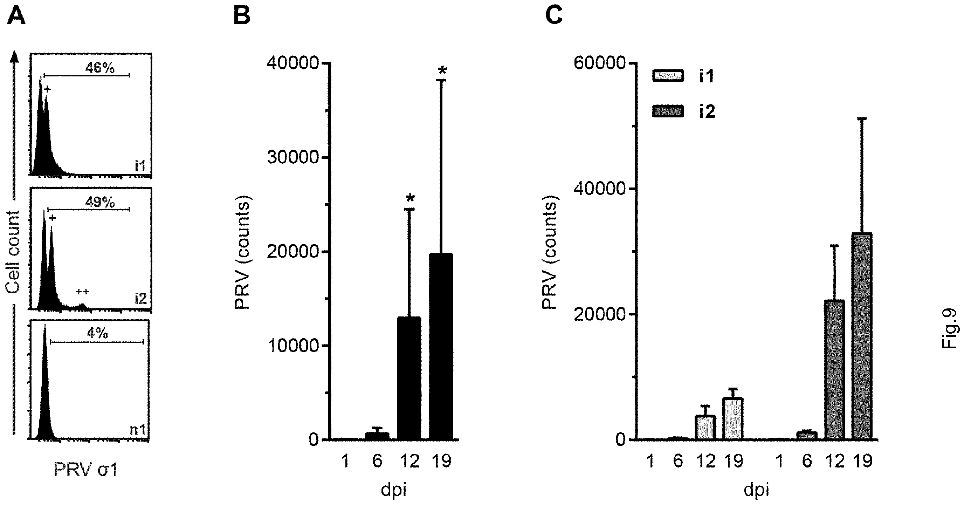

Figure. 9 Viral load by qPCR produced by *ex vivo* infection of PRV. (A) Isolated RBC from *in vivo* infected fish i1 and i2 and naïve fish n1 screened for PRV o1 protein by flow cytometry before initiation of the *ex vivo* infection. PRV low positive (+) and high positive (++) population are indicated in i1 and i2. 50 000 cells were counted for each sample. (B) Mean PRV count (and SD) detected by qPCR during *ex vivo* infection experiment presented for all samples (n=6). (C) In addition, mean PRV count (and SD) and shown by separating native RBC infected with i1 lysate (n=3) and i2 lysate (n=3). Data were analyzed using Wilcoxon matched pairs signed rank test. * p<0.05.

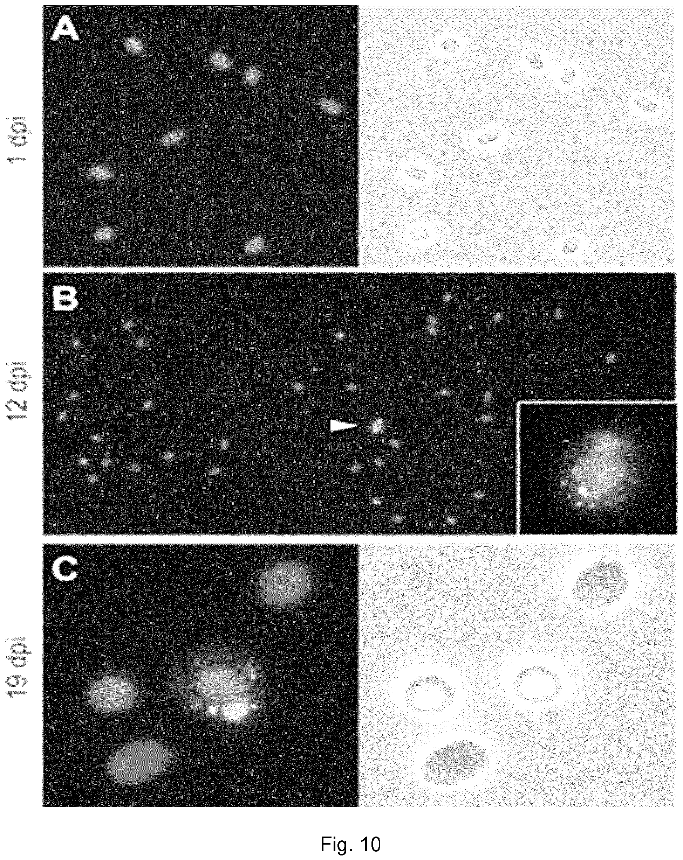

Figure 10. Immunofluorescence microscopy of RBC during the *ex vivo* infection experiment. Fluorescent labeling of the PRV o'1-protein in erythrocytes (green) in *ex vivo* infected RBC during the experiment. Cell nucleus stained with propidium iodide (red). No positive cells were observed at 1 dpi (A) or 6 dpi. At 12 dpi (B) and 19 dpi (C) large amounts of PRV o'1-protein was observed in a few single cells as cytoplasmic inclusions (green) which were also visible in phase contrast. Note the rounded cell and nucleus of PRV positive cells.

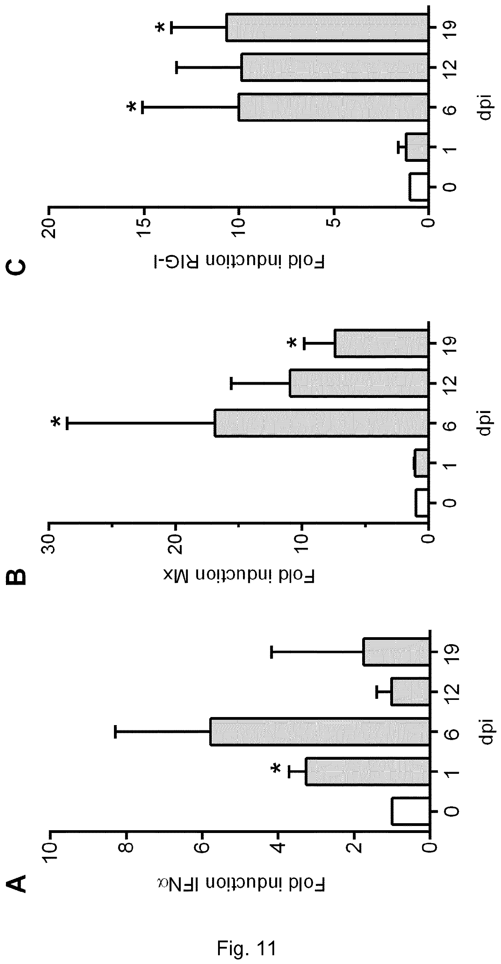

Figure 11. Antiviral responses to *ex vivo* PRV infection. RBC from three naïve fish n1-n3, infected with erythrocyte lysate from two different PRV-infected fish i1-i2 were analyzed for antiviral gene expression by RT-qPCR prior to infection, and at day 1, 6, 12 and 19 following introduction of PRV. Levels were normalized for the expression of the elongation factor (EF) 1α reference gene. Mean fold induction relative to 1 dpi for each of the six cultures is shown for IFNα (A), Mx (B) and RIG-I (C). Data were analyzed using Wilcoxon matched pairs signed rank test. * p<0.05.

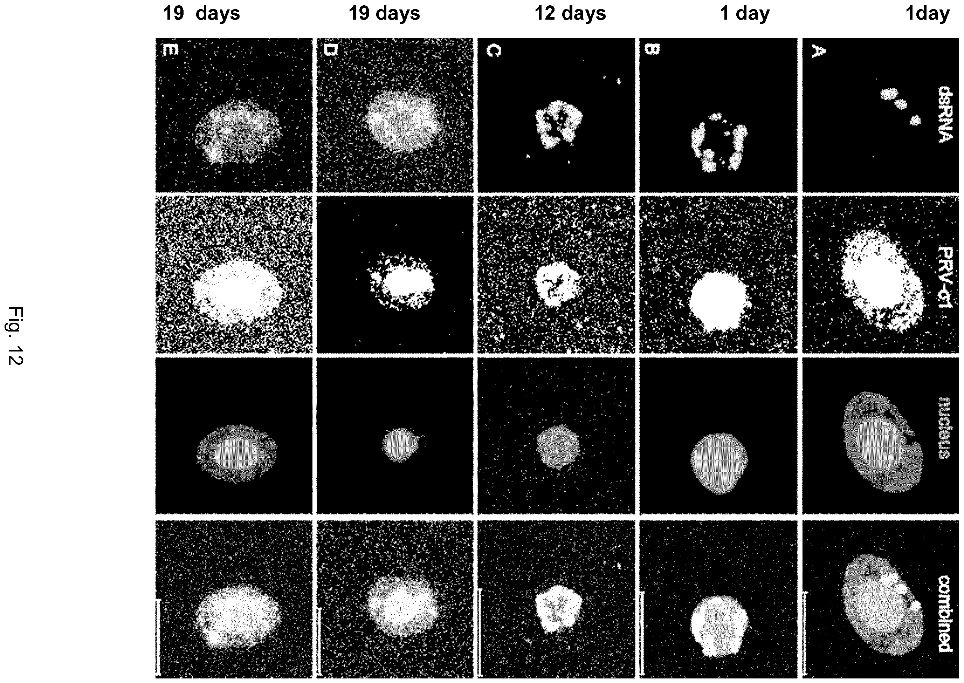

Figure 12. Characterization of RBC and inclusions isolated from *in vivo* PRV infected RBC. Cultured RBC isolated from *in vivo* infected fish i1 and i2 were stained with anti-double stranded RNA (dsRNA, green), anti-PRV-o'1 (blue) and studied by confocal microscopy. The nuclei were stained with propidium iodide (red). (A) and (B) show typical PRV o1 protein and dsRNA positive cells found after 1 day in culture; (C) strongly stained cell 12 dpi, and (D) and (E) typical cells found after 19 days in culture. Pictures show combined signals from confocal microscopy at 63x magnification, scale bare = 10 µm.

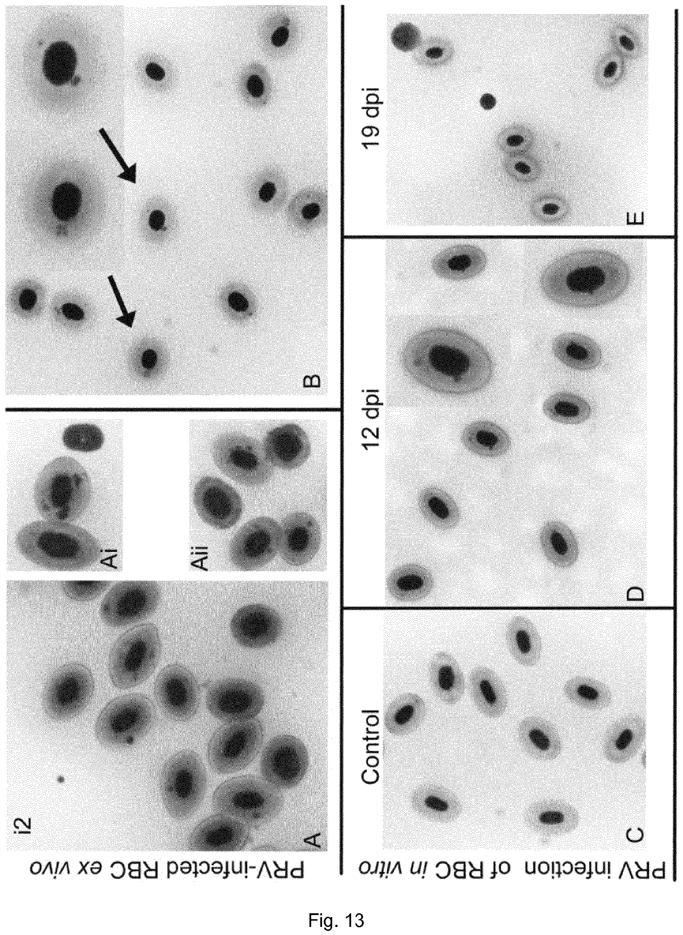

Figure 13. Pinacyanol chloride staining of PRV infected RBC. Pinacyanol chloride staining of RBC infected *in vivo* with PRV, isolated from infected fish i2 and kept in culture. Cytoplasmic inlcusions of various size and numbers were observed (A, B). Pinacyanol chloride staining of RBC *ex vivo* infected with PRV (C-E). No inclusions observed in RBC prior to infection (C). At 12 days post infection, cytoplasmic inclusions were observed (D). At 19 days post infection ghost cells were detected (E).

### Abbreviations

HSMI; heart and skeletal muscle inflammation
IF; Immunofluorescence
PRV; Piscine orthoreovirus, also named piscine reovirus
RBC; red blood cells
RT-qPCR; reverse transcription-quantitative polymerase chain reaction
MGBNFQ; Minor Groove Binding Non-Fluorescence Quencher
TAMRA; tetramethylrhodamine
FAM; 6-carboxyfluorescein

### DEFINITIONS

*Ex vivo* as referred to herein means an event which takes place outside an organism. *Ex vivo* may e.g. refer to experimentation and/or measurements done in or on tissue in an artificial environment outside the organism with the minimum alteration of natural conditions. In the context of the present document, the term *ex vivo* may be interchangeably used with the term *in vitro.*

*Salmonidae* is a family of ray-finned fish placed in the order *Salmoniformes.* These may also be referred to as salmonids. *Salmonidae* includes salmon, trout, chars, freshwater whitefishes and graylings.

A cell culture as referred to herein may be explained as the process by which cells are grown under controlled conditions, generally outside of their natural environment. Conditions for such cell cultures are exemplified in the experimental section.

Co-cultivation as referred to herein, refers to the cultivation of different cell populations in close proximity in the same cell culture environment. However, co-cultivation as used herein also refers to the cultivation of a cell population in close proximity with a material comprising an orthoreovirus. In the method disclosed in the present document, co-cultivation thus involves mixing erythrocytes substantially free from orthoreovirus with a material comprising the orthoreovirus of interest for propagation, such as cells infected with orthoreovirus or any other material containing a source of orthoreovirus, such as a cell supernatant comprising orthoreovirus, isolated virus etc., (the mixture thus obtained herein being referred to as a mixed culture, see step a)). Thereafter the erythrocytes substantially free from orthoreovirus are incubated with the material comprising the orthoreovirus of interest for propagation (see step b) in the method disclosed herein. Co-cultivation as referred to herein thus involves both the cultivation of erythrocytes substantially free from orthoreovirus with cells infected with orthoreovirus and/or cultivation of erythrocytes substantially free from orthoreovirus with any other material containing a source of orthoreovirus (such as a cell supernatant comprising the virus, isolated virus etc.).

Erythrocytes which are substantially free from orthoreovirus refer to erythrocytes which may contain small amounts of orthoreovirus, which small amounts may not be detectable by methods used in the art. They may also be fully free of the virus.

"Nucleated erythrocytes" and the like refer to erythrocytes which have a cell nucleus that allows for viral replication. Vertebrate erythrocytes, except for mammalian erythrocytes, generally contain a cell nucleus and are therefore suitable for use in accordance with the present document. For example, nucleated piscine erythrocytes, such as erythrocytes from *Salmonidae,* such as salmon erythrocytes or erythrocytes from trout, rainbow trout, chars, freshwater whitefishes and graylings, are suitable for use in accordance with the different aspects of the present document.

"Any other material containing a source of orthoreovirus" may e.g. be material from another tissue suitable to obtain orthoreovirus from blood, organs, cells and secretes from fish, such as Salmonids. Also, a supernatant obtained from lysed cells (such as erythrocytes) infected with orthoreovirus may be used as a source of orthoreovirus. Such a supernatant may be obtained by lysis, such as by freezing and thawing the cells followed by centrifugation (such as at about 700-900xg, such as about 800xg) of the cell lysate in growth medium and/or another solution, such as a buffer, to remove the cells and/or cell debris to provide a substantially cell free supernatant containing orthoreovirus. Any other material containing a source of orthoreovirus may also be the orthoreovirus as such, such as isolated orthoreovirus.
Herein, a piscine orthoreovirus may also be referred to as a piscine reovirus or PRV, hence the terms may be used interchangeably herein and refer to a piscine reovirus PRV.

Further herein, the term "propagating an orthoreovirus" and the like is intended to refer to the replication of the virus, i.e. the multiplication thereof which is shown herein to occur in erythrocytes, having a cell nucleus, in particular piscine erythrocytes, such as *Salmonidae* erythrocytes, such as salmon erythrocytes, generating multiple copies of the virus.
A vaccine or a vaccine composition, as mentioned herein, is intended to refer to a composition resulting in immunological prophylaxis in a subject to which the vaccine is administered. A vaccine composition induces an immune response and thus a long-acting immunity, to a specific antigen. In the present context an antigen is mainly intended to refer to an inactivated form of an orthoreovirus, or parts or fragments thereof which are still capable of generating an immune response in a subject.
By "identity" is in the context of the present document intended the extent to which two (nucleotide or amino acid) sequences have the same residues at the same positions in an alignment, expressed as a percentage. A local algorithm program may be used to determine sequence identity. Local algorithm programs, (such as Smith Waterman) compare a subsequence in one sequence with a subsequence in a second sequence, and find the combination of subsequences and the alignment of those subsequences, which yields the highest overall similarity score. Internal gaps, if allowed, are penalized.

Local algorithms work well for comparing two multidomain proteins, which have a single domain or just a binding site in common.
Methods to determine identity and similarity are codified in publicly available programs. Preferred computer program methods to determine identity and similarity between two sequences include, but are not limited to, the GCG program package (Devereux, J et al (1994)) BLASTP, BLASTN, and FASTA (Altschul, S.F. *et al* (1990)). The BLASTX program is publicly available from NCBI and other sources (BLAST Manual, Altschul, S.F. et al (1990)). Each sequence analysis program has a default scoring matrix and default gap penalties. In general, a molecular biologist would be expected to use the default settings established by the software program used. For example, by a nucleic acid sequence having e.g. 80% "identity" to a referred sequence is in the context of the present document intended a sequence wherein a total of 20% of the nucleic acid bases in the referred sequence have been substituted, deleted and/or added.

### DETAILED DESCRIPTION

The invention is as defined in the claims.

Orthoreoviruses are members of the *Reoviridae* virus family. Orthoreoviruses have a double stranded RNA genome and thus belong to group III viruses, which infects vertebrates. The closest relative to orthoreoviruses is the genus aquareoviruses (Nibert *et al.* 2013).
Herein, it is shown for the first time that blood and the red blood cell (erythrocyte) population is the main source of piscine reovirus (PRV) *in vivo,* and that red blood cells (erythrocytes) is a potent material for transferring virus between fish. Furthermore, as shown by flow cytometry, immunofluorescence staining and electron microscopy proves that PRV infects up to 50% of the Red Blood Cell (RBC) population and forms factory-like structures indicating replication. Using electron microscopy it is shown herein that these inclusions contained reovirus-like particles. Accordingly, it is concluded that PRV replicates in Atlantic salmon erythrocytes prior to infecting cardiomyocytes. These findings provide new information about HSMI pathogenesis, and show that PRV is an important factor of viral erythrocytic inclusions. This is a surprising finding as it was not previously known where the PRV virus replicates. Furthermore, this is the first demonstration of a reovirus replicating in nucleated erythrocytes.
Piscine reovirus (PRV) has been further characterized by Palacios G., *et al.* (3) and Markussen T. *et al.* (4).

The findings shown herein made it possible to construct an *ex vivo* method for propagating and isolating PRV from nucleated erythrocytes *ex vivo* as shown herein, as the orthoreovirus piscine reovirus (PRV) surprisingly was found to be able to replicate in such an environment generating large amounts of virus for further isolation, characterization and use. There has been no objective in the art to use erythrocytes isolated from a biological sample of *Salmonidae,* i.e. a blood sample containing erythrocytes, grown *ex vivo* in culture in the manner as disclosed herein, thereby allowing the production of large amounts of virus. This as it was not expected that such an *ex vivo* model was suitable. Fish viruses and mammalian viruses have commonly been found associated with erythrocytes by surface binding, but replication in erythrocytes has not been demonstrated for viruses infecting *Salmonidae.* Also, propagation in erythrocytes to produce large amounts of virus *ex vivo* could not be expected.

Hence, the present method has surprisingly been shown to provide a way of obtaining large amounts of piscine reovirus which can then, once inactivated, become of further use in a vaccination product suitable for vaccinating *Salmonidae* against HSMI (Heart and skeletal muscle inflammation).

Accordingly, it is surprisingly shown that erythrocytes are a major target cell for PRV replication. Further, that it was possible to use these findings to generate an *ex vivo* model for producing large amounts of piscine reovirus was not evident e.g. in view of the sensitivity of the system. In addition, there has been no motivation to use erythrocytes *ex vivo* for performing such a measure e.g. in view of that mammalian RBC lacks nucleus and does not provide any means for viral replication.
Based on the earlier detection of PRV in erythrocytes and leukocyte-like cells and common amino acid motifs with the MRV hemagglutinating cell attachment protein the hypothesis put forward was that there is a cell associated viremia in a PRV infection which is important for the pathogenesis. In two subsequent PRV challenge experiments, the viral loads in blood, plasma and erythrocytes were assessed in the different phases of infection. The intracellular presence of high loads of PRV was found in more than 50% of the erythrocyte population in some individual fish as demonstrated by flow cytometry, immunofluorescence, confocal microscopy and electron microscopy. Accordingly, it is surprisingly shown that erythrocytes are a major target cell for PRV replication, which was not expected as PRV has earlier been shown to be present in several tissues but still not in large amounts. Further, that it was possible to use these findings to generate an *ex vivo* model for producing large amounts of PRV was not evident. In addition, there has been no motivation to use erythrocytes *ex vivo* for performing such a measure e.g. in view of that mammal blood lacks nucleus not providing any means for replication.
Earlier attempts of propagating PRV from cell lines have not been successful.
This newly identified viral propagation system is also of great importance for research aiming to characterize the virus, the viral infection mechanisms, and develop methods to modulate viral spread. A system to grow the virus will also allow development of recombinant viruses and provide a system for the characterization of potential differences in virulence in PRV variants that that may show up worldwide. The present method for propagating and/or detecting PRV may have a large impact on the propagation and/or detection of other piscine orthoreoviruses or piscine reoviruses. Accordingly, by a method as disclosed herein, it is possible to produce an inactivated virus vaccine, i.e. a vaccine composition containing an inactivated PRV which may be used for vaccinating fish, such as *Salmonidae,* such as salmon, against disease associated with PRV. Such a disease is for example Heart and skeletal muscle inflammation (HSMI).
Based on the surprising findings presented herein, the present document discloses an *ex vivo* method for propagating and isolating PRV, said method comprising *ex vivo* propagating PRV in nucleated piscine erythrocytes, followed by isolating the PRV.

The orthoreovirus according to the different aspects of the present document is piscine reovirus (PRV).

Erythrocytes which may be used in the different aspects of the present document are any nucleated piscine erythrocytes, i.e. any piscine erythrocytes which have a cell nucleus allowing for viral replication. For example, nucleated piscine erythrocytes, such as erythrocytes from *Salmonidae,* such as salmon erythrocytes, or erythrocytes from trout, chars, freshwater whitefishes and graylings, are suitable for use in accordance with the different aspects of the present document. However, erythrocytes of salamanders of the *Batrachoseps* genus, fish of the *Maurolicus* genus and closely related species, and mammals lack cell nucleus and may therefore not be used in the different aspects of the present document.

A method for propagating and isolating a PRV according to the present document may comprise the steps of:
a) co-cultivation of nucleated piscine erythrocytes, substantially free from reovirus, with nucleated piscine erythrocytes infected with PRV and/or any other material containing a source of PRV, to obtain a mixed culture;
b) incubating the mixed culture obtained in step a),
c) removing erythrocytes infected with PRV from said mixed culture of step b),
d) optionally repeating the procedure of steps a) to c), and
e) isolating PRV from the erythrocytes of step c).
In particular, there is provided an *ex vivo* method for propagating and isolating piscine reovirus (PRV), wherein said method comprises the steps of: a) co-cultivation of *Salmonidae* erythrocytes substantially free from piscine reovirus with *Salmonidae* erythrocytes infected with piscine reovirus and/or any other material containing a source of piscine reovirus to obtain a mixed culture; b) incubating the mixed culture obtained in step a), c) removing erythrocytes infected with piscine reovirus from said mixed culture of step b), d) optionally repeating the procedure of steps a) to c), and e) isolating piscine reovirus from the erythrocytes of step c).
Erythrocytes may be isolated from a *Salmonidae* blood sample by standard procedure, such as described in the experimental section.
For example, the procedure may be performed by isolating nucleated erythrocytes from a suitable source, such as adult Atlantic salmon (such as approx. 2 kg), confirming uninfection with orthoreovirus, e.g with PRV, by RT-qPCR (spleen). Erythrocytes may thereafter be washed, counted and dissolved to about 5 mill/ml in a suitable medium, such as Leibovitz L15 media supplemented with fetal calf serum (10%), and a suitable antibiotics, such as gentamicin (e.g. ca 50 µg/ml). Cells may thereafter be plated e.g. in a 24 well plate with about 5 mill cells/well and kept on gentle agitation at about 12-15°C in a non-CO₂ incubator, e.g. with 200 rpm agitation. Freshly isolated erythrocytes (e.g. from three separate fish) which are infected with PRV (see Challenge experiment # 2, cohabitants, 6WPC) are added (about 0.1 mill cells/well), and cells may thereafter be incubated for up to about 13 days (at about 12-15°C). About 250 µl cells may be harvested per time-point in sterile Eppendorff tubes and thereafter centrifuged (e.g. 750 x g, 5 min). The media is thereafter discarded, and RNA may be isolated from the erythrocyte pellet. The previous is an example of a procedure, but the disclosure is not intended to be limited thereto.
As mentioned, the method may be repeated so that a suitable amount of the piscine reovirus is obtained after the *ex vivo* culturing method has been performed for an appropriate amount of times, such as exemplified herein.
The incubation of step b) of a method as disclosed herein may be performed for about 14 days, but is not limited thereto. It may e.g. be performed for about 4 days or more, such as about 4-45 day, such as 4-30 days, such as 5-30 days, such as 5, 6, 7, 8, 10, 11, 12, 13 or 14 days. The incubation may be performed at a temperature of about 5-30°C, such as 5-20°C, 10-18°C, 12-18°C or 12-15°C, such as about 10, 11, 12, 13, 14 or 15°C. Any media suitable for maintaining erythrocytes may be used, such as Leibovitz L15 media supplemented with fetal calf serum (such as about 2-10% e.g. 2, 3, 4, 5, 6, 7, 8, 9 or 10%). A suitable antibiotics, such as gentamicin (e.g. ca 50 µg/ml), may also be added to the medium. Furthermore, the procedure of steps a) to d) may be repeated at least 2 times, such as about 2-10 times. As mentioned, the procedure may be repeated to generate an amount of the PRV which is sufficient for further use of said virus. It should be noted that the procedure may be monitored by measuring the virus content in the cell culture, e.g. by using RT-qPCT, to thereby decide whether to proceed with the culturing or if enough virus may currently be obtained therefrom.

In step c) erythrocytes infected with PRV are removed from the incubation media. This may e.g. be performed by centrifuging, such as by gradient centrifugation, or filtrating the erythrocytes to separate them from the incubation media.
The isolation of step e) may e.g. be performed isolating RNA from the erythrocytes removed in step c). For such RNA isolation any standard technique for RNA isolation may be used, such as RNeasy® from Quiagen.

A source of PRV to be used for co-cultivation with erythrocytes substantially free from reovirus may be in the form of an inoculum prepared from cells infected with PRV. The inoculum may be prepared by lysing cells infected with PRV and collecting a cell free supernatant thereof comprising the PRV.

Such preparation of an inoculum comprising a PRV may be prepared by collecting PRV infected blood in heparinized tubes, centrifuging, e.g. at about 2000g for about 10 min at cold temperature, such as about 4°C, and removing the blood plasma. The blood may then be diluted, such as about 5-20 times, e.g. about 10 times in a suitable buffer, such as PBS, before the cells are lysed. The cells may be lysed by any method suitable for lysing cells. Cell lysis may e.g. be effected by a freeze/thawing cycle, e.g. by alternate freezing and thawing the sample, such as for three times. The temperature used for freezing may e.g. be between -10 to -85°C, such as about -80°C. After the freeze/thawing cycle is finished, the sample may be centrifuged, such as at about 2000g for 5 min at 4°C, to obtain a supernatant. The supernatant is thereafter collected and can be used as an inoculum. An inoculum comprising PRV may also be prepared by sonication of cells infected with PRV. Such a sonication is preferably carried out on ice. The sonication may e.g. comprise repetitive pulses, such as for 10 s, of sonication alternated with a rest in between. The number of pulses may e.g. be 10. The time for rest between the pulses may e.g. be about 30 s. After the sonication is finished, the sample may be centrifuged, such as at about 2000g for 5 min at 4°C, to obtain a supernatant. The supernatant is thereafter collected and can be used as an inoculum. The amount of inoculum to use will of course depend on the amount of virus for infection that is desirable, but e.g. an amount of 10-500 µl of an inoculum prepared according to the freeze/thawing or sonication method above may be used, such as about 100 µl.
In the present context, said *Salmonidae* may be a salmon or a rainbow trout or trout, chars, freshwater whitefishes and graylings.
The present document is also directed to an *ex vivo* method of preparing a vaccine composition, said method comprising *ex vivo* propagating a PRV in nucleated piscine erythrocytes, followed by isolating the PRV and thereafter preparing a vaccine composition containing said inactivated PRV. Examples of nucleated erythrocytes in such a method are as disclosed elsewhere herein.
Such an *ex vivo* method of preparing a vaccine composition may comprise the steps of:
a) co-cultivation of nucleated piscine erythrocytes, substantially free from reovirus with nucleated piscine erythrocytes, infected with PRV and/or any other material containing a source of PRV to obtain a mixed culture;
b) incubating the mixed culture obtained in step a),
c) removing erythrocytes infected with PRV from said mixed culture of step b),
d) optionally repeating the procedure of steps a) to c), and
e) isolating PRV from the erythrocytes of step c),
f) inactivating the PRV obtained in step e) and thereafter preparing a composition containing said inactivated PRV.
Such an *ex vivo* method of preparing a vaccine composition may for example comprise the steps of a) co-cultivation of *Salmonidae* erythrocytes substantially free from piscine reovirus with *Salmonidae* erythrocytes infected with piscine reovirus and/or any other material containing a source of piscine reovirus to obtain a mixed culture; b) incubating the mixed culture obtained in step a), c) removing erythrocytes infected with piscine reovirus from said mixed culture of step b), d) optionally repeating the procedure of steps a) to c), and e) isolating piscine reovirus from the erythrocytes of step c) followed by inactivating the piscine reovirus obtained in step e) and thereafter preparing a composition containing said inactivated piscine reovirus.

Inactivation of the PRV may be performed in any suitable manner known in the art, such as by inactivation using solvents or detergents, heat inactivation or inactivation by exposing the virus to acidic pH. Steps a)-e) in such a method for preparing a vaccine composition are performed as disclosed elsewhere herein.

Accordingly, a vaccine composition comprising an inactivated form of the piscine reovirus may be prepared according to measures known in the art and suitable excipients may be added to said composition.

There is also provided herein a method wherein said vaccine composition containing an inactivated piscine reovirus obtained through an *ex vivo* method as disclosed herein is to be used for the treatment and/or prevention of Heart and Skeletal Muscle Inflammation (HSMI). Also, the vaccine composition may comprise one or more of other orthoreovirus(es) or other antigenic agent(s). There is also provided the use of a vaccine composition containing an inactivated piscine reovirus obtained through an *ex vivo* method as disclosed herein, in the manufacture of a medicament for the treatment and/or prevention of Heart and Skeletal Muscle Inflammation.
There is also provided herein an *ex vivo* method for diagnosing Heart and Skeletal Muscle Inflammation (HSMI) or the presence of PRV virus in fish of the species *Salmonidae,* said method comprising: a) isolating *Salmonidae* erythrocytes from a biological blood sample from said fish of a species of *Salmonidae,* b) detecting the presence of piscine reovirus (PRV) in said *Salmonidae* erythrocytes obtained from step a). In such a method, the *Salmonidae* erythrocytes may be isolated as disclosed and exemplified elsewhere herein. Such detection may e.g. be performed by RT-qPCR. The primers and/or probes disclosed in Table 2 may be used for such detection.
In an *ex vivo* method as provided herein said *Salmonidae* may be salmon. Detecting the presence of piscine reovirus in erythrocytes is an indication of, or the possible development of, Heart and Skeletal Muscle Inflammation (HSMI) in a fish of a species of *Salmonidae.*

The present document is also directed to the use of nucleated piscine erythrocytes for propagating PRV. Suitable nucleated erythrocytes and PRV for such use are as disclosed elsewhere in this document. An example of a use is the use of *Salmonidae* erythrocytes infected with piscine reovirus for propagating piscine reovirus.

The present disclosure is further illustrated by way of an experimental section, but is not intended to be limited thereto.

### EXPERIMENTAL SECTION

### Example 1

### Materials and methods

### Experimental fish and rearing conditions.

Two subsequent PRV challenge experiments were conducted (VESO Vikan, Norway). Both trials were performed using unvaccinated seawater adapted Atlantic salmon. The fish were kept in tanks supplied with filtered 25‰ salinity seawater, 12°C ± 1°C and with a 12:12 h light/dark regime. The fish were acclimatized for 2 weeks prior to challenge, fed according to standard procedures and anesthetized by bath immersion (2-5 min) in Metacain (1 g/10 I water buffered with 1 g NaHCO₃) (Norsk Medisinaldepot; Oslo, Norway) before handling and killed by a blow to the head.

**Challenge experiment # 1.** In the first experiment 50 shedders and 50 cohabitants with an initial average weight of 50 g were used. The inoculum consisted of tissue homogenate (heart, spleen and head-kidney) from fish from a field outbreak of HSMI and contained a high load of PRV as determined by RT-qPCR. The inoculum was confirmed free of other commonly known salmon pathogens. The shedders were anesthetized, given 0.1 mL inoculum by intraperitoneal (i.p.) injection, marked by shortening the outer left maxilla and put in a tank containing 50 naïve fish (cohabitants). Six fish of the cohabitant group were sampled every second week starting at 6 weeks post challenge (wpc) ending at 14 wpc. In addition 3 fish were sampled at 4 wpc to test for transmission of PRV. Control samples from 6 fish were collected before initiation of the experiment. At each sampling, peripheral blood from the caudal vein was collected in heparinized vacutainers, and tissue from heart, skeletal muscle, spleen and head kidney were sampled in RNAlater (Life Technologies, Carlsbad, CA) and used for RT-qPCR analysis. Parallel samples from the same organs were harvested in 10% phosphate-buffer formalin, embedded in paraffin and used for histologic analysis.

At 9 wpc blood from 15 fish from the cohabitant group was sampled, pooled and centrifuged. After removing the plasma the blood pellet was diluted 1:4 in phoshate-buffered saline (PBS) and stored at -80°C. The pooled sample was used as inoculum in challenge experiment # 2.

**Challenge experiment # 2.** In the second challenge experiment, 60 shedders and 60 cohabitants with an initial average weight of 80 g were used. The blood pellet prepared in experiment # 1 was thawed, diluted 1:3 in PBS and used as challenge material (Marie L: Ct-verdi for PRV). Inoculation and labeling of the shedders were performed as in Challenge #1. Six fish from the cohabitant group were sampled weekly from 2 wpc until 8 wpc. Control samples from six fish were sampled before initiation of the experiment. Blood, heart and spleen tissues were sampled as described in Challenge # 1. Samples for RT-qPCR were taken from heparinized blood as well as the plasma fraction collected after centrifugation at 1000 x g for 5 min. The remaining blood sample was used for isolation of red blood cells (RBC), which were subsequently analyzed by RT-qPCR, flow-cytometry, confocal microscopy and transmission electron microscopy (TEM).

**RNA isolation.** Total RNA was isolated from the tissues stored in RNAlater by homogenization in QIAzol (Lysis Reagent (Qiagen, Hilden, Germany)) using 5 mm steal beads and TissueLyser II (Qiagen). After addition of chloroform and centrifugation, the aqueous phase was collected and mixed with one volume of 70% ethanol before continuing with the RNeasy Mini spin column as described by the manufacturer. The same method was used for isolation of total RNA from the heparinized blood samples and isolated RBC using an input of 10 µL blood and 1 x10⁷ RBC, respectively. For the plasma samples, a combination of Trizol LS (Invitrogen Life Technologies) and RNeasy mini kit was used. Briefly, 50 µL plasma was mixed and incubated with Trizol LS before adding chloroform, separating the phases by centrifugation, and subsequently RNeasy mini kit was used as recommended by the manufacturer. The RNA isolated from 50 µL plasma was eluted in 50 µL RNase-free water. RNA was quantified using a NanoDrop ND-1000 spectrophotometer (NanoDrop Technologies).

**RT-qPCR.** The Qiagen OneStep kit (Qiagen) was used for RT-qPCR. A total of 5 µL RNA (20 ng/µL) from tissue samples or 5 µL RNA from plasma samples, equivalent to 5 µL plasma, were denaturated at 95 °C for 5 min. RT-qPCR were performed using the conditions: 400 nM primer, 300 nM probe, 400 nM dNTPs, 1.26 mM MgCl₂, 1:100 RNase Out (Invitrogen) and 1X ROX reference dye with the following cycle parameters: 30 min at 50 °C, 15 min at 94 °C, 40 cycles of 94 °C/15 s, 54 °C/30 s and 72 °C/15 s in a Mx3005P (Stratagene). The samples were run in duplicates, and a sample was defined as positive if both parallels had a Ct<35. The sequence of the primers and probe used targeted PRV gene segment S1 were: S1Fwd TGCGTCCTGCGTATGGCACC (SEQ ID NO:1); S1Rev GGCTGGCATGCCCGAATAGCA (SEQ ID NO:2); S1probe (FAM)-ATCACAACGCCTACCT-MGBNFQ (i.e. FAM-SEQ ID NO:3-MGBNFQ).

**Histopathology.** Slides from the formalin fixed and paraffin embedded heart material collected from Challenge # 1 and 2 was hematoxylin-eosin (HE) stained and evaluated for histopathological changes by light microscopy.

**Isolation of red blood cells (RBC).** RBC were isolated from the heparinized blood collected in Challenge # 2 by using a previously described Percoll gradient with minor modifications (27). In short, blood was diluted 1:10 in distilled PBS (dPBS) and a discontinuous Percoll gradient was prepared using 1.07 g/mL (49%) and 1.05 g/mL (34%). The diluted blood was layered onto the gradient and centrifuged at 2000 x g for 20 min at 4 °C. The RBC were collected from the bottom of the gradient; however a variable amount of erythrocytes was present in the 34%-49% interface. In order to collect the remaining erythrocytes, the interface was relayered onto a fresh 34%-49% gradient and centrifuged at 500 x g for 10 min at 4 °C. The RBC from the bottom of the gradient was collected, pooled with the erythrocytes from the first gradient and washed twice in PBS. The cells were counted using Countess (Invitrogen) and resuspended in PBS to a final concentration of 5 x 10⁶ cells/mL. Blood smears stained with Diff Quick confirmed that the isolated cells could be identified as erythrocytes.

**Flow cytometry.** In order to detect PRV positive cells, the isolated RBC from 0, 4, 5, 6, 7 and 8 wpc were stained using a polyclonal antibody against putative PRV outer capsid protein o1 (Anti-o'1, #K275) (18), running parallel samples for surface and intracellular labeling. Briefly, 0.5 x106cells per well were plated into 96 well plates and washed in staining buffer (PBS + 1% BSA + 0.05% azide). Cells were surface stained with primary antibody Anti-σ1 #K275 (1:10 000) for 30 min and secondary FITC conjugated anti-rabbit IgG (Molecular probes) (2mg/mL diluted 1:800) for 30 min using staining buffer in all washes and dilutions. Prior to intracellular staining the cells were fixed and permeabilized by incubation in Cytofix/Cytoperm (Becton Dickinson) for 15 min on ice, and the same primary and secondary incubations described above but using Perm/wash (BD) in the dilutions and washing steps. All incubations were performed on ice and the corresponding zero serum (Anti-σ1 Zero #K275) was used as negative control serum. The cells were read on Gallios Flow Cytometer (Beckman Coulter) counting 50 000 cells per sample, and the data were analyzed using the Kaluza software (BD). Due to slight variation in the background staining, the flow charts were gated individually discriminating between negative and positive peaks.

**Immunofluorescence microscopy and confocal microscopy.** Selected samples of the intracellularly stained RBC were prepared for immunofluorescence and confocal microscopy. The nuclei were stained with propidium iodide and the cells were mounted to glass slides using Fluoroshield (Sigma-Aldrich) and cover slips. Non-saturated images were captured by a Plan-Apochromat 63/1.4 oil objective in a laser scanning confocal microscope (Zeiss Axiovert 200M fluorescent inverted microscope, equipped with a LSM 510 laser confocal unit and 488 nm argon laser, 546 nm and 633 nm helium/neon laser Z). In addition, selected samples were stained with propidium iodide as described above and the cell suspension was transferred to Countess chamber slides (Invitrogen). Pictures were taken using an inverted fluorescence microscope (Olympus IX81) at 20x and 40x magnification.

**Transmission electron microscopy (TEM).** Isolated RBC from infected fish were fixed overnight at 4°C in PBS with 1.25% glutaraldehyde and 2% paraformaldehyde, washed twice in the PBS and three times in cacodylate buffer (0.1 M, pH 6.8) The cells were postfixed with 1% OsO₄ for 1 h at 4°C and washed several times with cacodylate buffer. The cells were then dehydrated through an ethanol series (70, 90, 96 and 100%) and embedded in LR-White resin. Thin sections were cut on an ultra microtome (LEICA EM UC 6). The sections were stained with 4% aqueous uranyl acetate and 1 % KMNO₄ for 10 min, then washed intensively in freshly distilled water. The sections were examined in a FEI MORGAGNI 268, and photographs were recorded using a VELETA camera.

**Statistical analysis.** All statistical analyses were performed with GraphPad Prism 6.0 (GraphPad Software inc., USA).

### Results

**High PRV load in blood during infection.** The RT-qPCR results from Challenge # 1 revealed a high viral load in heparinized whole blood samples. Piscine reovirus was first detected in blood at 6 wpc, and the amount of virus peaked at 8 wpc with a mean Ct-value of 18.6 (±0.7). High viral loads were present in blood until the end of the study (Fig. 1A). The mean Ct-values of blood were compared to heart and skeletal muscle, which are the main organs for histopathological changes during HSMI (3), using unpaired t-test. Significantly lower amounts of virus were found at the early (6 and 8 wpc) and late (14 wpc) phases in the heart (p<0.05), and at all time points in the skeletal muscle (p<0.05) (Fig. 1A). When comparing blood to spleen and head-kidney, the organs known to contain most virus during an infection (19), levels were approximately the same (Fig. 1B). However in the later phase (14 wpc) there was a significantly higher viral load in the spleen (p<0.01). Histopathological changes in heart and skeletal muscle consistent with an HSMI diagnosis were first observed at 10 wpc in three out of six fish at 10 wpc.

In order to further assess the differences between blood and tissue PRV levels statistically, the relative Ct-values in each individual fish at each time point were analyzed using a paired t-test. The results showed that the relative amounts of virus in both heart and skeletal muscle samples were significantly lower than those of blood in the same individual at all time-points (p<0.05) (Fig. 1C). The relative PRV load in spleen and head-kidney increased compared to blood during the infection. At 6 wpc, there was a significantly lower PRV load in spleen and head-kidney compared to blood (p<0,01), whereas in the later phase (10 - 14 wpc) the viral loads in spleen were significantly higher (p<0.05), and the same tendency was seen for head-kidney (significantly higher only at 10 wpc, p<0.05). All Ct-values from Challenge # 1 are listed in table S1.
**Blood cell pellet is a highly effective challenge material.** A blood cell pellet prepared from Challenge # 1 was used as inoculum in Challenge # 2, and again, high viral loads were detected in blood (Fig. 2A). PRV was first detected in three out of six fish at 4 wpc; i.e. two weeks earlier than in Challenge # 1, and at 5 wpc all sampled fish were PRV positive by RT-qPCR. The maximum viral load in blood appeared two weeks earlier compared to Challenge # 1, and plateaued at 6 wpc with a mean Ct-value of 16.5 (±0.7); which was significantly higher than viral loads at the same time point in Challenge # 1 (unpaired t-test p<0.001).
The amount of virus in blood was compared to the spleen, and as observed in Challenge # 1, PRV loads of blood were significantly higher to that of the spleen in the early phases of the infection (5-7 wpc) (p-value <0.01) (Fig. 2B). Histopathological changes in heart and skeletal muscle consistent with an HSMI diagnosis were first observed at 7 wpc in four out of six fish (Fig. 3) and in all sampled fish at 8 wpc.
**High PRV load in RBC.** PRV was first detected in RBC from Challenge # 2 by RT-qPCR at 4 wpc and plateaued about 6 to 7 wpc (18.5±2.9 and 17.5±2.9 respectively), thus following the same pattern observed in blood (Fig. 2A). These results indicated that RBC is an important virus reservoir during PRV infection. Plasma also contained a substantial amount of virus, partly explaining the slightly higher level of virus in whole blood compared to RBC. All Ct-values from Challenge # 2 are listed in table S2.
**Large population of PRV positive RBC detected by flow cytometry.** The isolated RBC from Challenge # 2 were stained using a polyclonal antibody against the putative PRV capsid protein o1, and both surface and intracellular staining was performed. Cells were gated according to their size and granularity to include only intact cells (Fig. 4A). Samples from 0 wpc were used as negative controls indicating the background fluorescence signal detected in PRV-negative samples (Fig. 4B). A PRV positive erythrocyte population was first observed at 5 wpc in two out of six individuals (F55, F56) seen by a peak in fluorescence signal for both the intracellular and surface staining. The majority of virus protein was detected intracellularly and a large, distinct population of PRV positive RBC (up to 43% positive cells in F56) could be observed (Fig. 4C). As shown by RT-qPCR of RBC the positive individuals also contained distinctly higher viral loads compared to the rest of the group (Table 1). PRV was also detected on the surface of lower numbers of RBC of the same individuals. The surface staining from challenge #1 is shown.

At 7 wpc, PRV positive erythrocytes were observed in four out of five individual fish and large populations of RBC (25 up to 51 %) were positive (Fig. 4C). The large majority of the virus was present intracellularly. In contrast to the 5 wpc samples, the positive cells at 7 wpc could be divided in two separate populations showing low positive and high positive staining for PRV o1. The low positive population was comparable in intensity to the single PRV population detected at 5 wpc, indicating a development of cells containing high PRV levels over time (Fig. 4C). At 8wpc large populations of PRV positive RBC were detected by intracellular staining in all six individuals, similar to the results obtained at 7 wpc.
The results from the flow cytometry analysis correlated with the RT-qPCR results, as individual fish with Ct-values below 20 were consistently detected as positive in the flow analysis at 5, 7 and 8 wpc (Fig. 4C, Table 1). Samples with Ct-values above 20 appeared to be below the sensitivity threshold of the flow cytometry analysis, exemplified by F74 at 7 wpc (Fig. 4C). At 4 wpc all the RBC samples had Ct-values above 20 producing only a background signal in flow cytometry, whereas at 8 wpc all samples had Ct-values below 20 and were positive by flow cytometry (Fig. 4C).

Throughout the study there was a tendency that PRV positive cells with high fluorescence intensity had an increased side scatter in flow cytometry (Fig. 4D). The negative population and high PRV positive population was individually gated in each fish and their mean side scatter was compared using wilcoxon test. The high PRV positive populations at 7 wpc and 8 wpc had a significantly higher side scatter compared to the negative population (p<0.05) indicating that highly PRV positive red blood cells are more granular.

**Viral cytoplasmic inclusions detected in RBC.** In order to study the subcellular localization of PRV, selected RBC samples stained intracellularly for PRV o1 were prepared for immunofluorescence- and confocal microscopy. The PRV positive erythrocytes were identified in the immunofluorescence microscope by granular staining in the cytoplasm (Fig. 5A). The positive staining was observed as inclusions that varied in size and number from a few large inclusions to multiple smaller inclusions, or a more diffuse granular staining throughout the cytoplasm (Fig. 5B). The morphology of the positive red blood cells varied from typical mature erythrocytes with ellipsoidal shape and long nucleus, to more circular cells resembling immature erythrocytes (polychromatocytes). The PRV positive staining was confirmed to be of cytoplasmic origin by confocal microscopy, and the viral inclusions were primarily detected in the perinuculear region, but also found scattered in the cytoplasm (Fig. 5C). Cytoplasmic inclusions were also observed in some RBC by phase contrast microscopy, and these inclusions co-localized with the immunofluorescent staining of PRV (Fig. 5D).

**Reovirus-like particles observed in TEM.** The TEM analysis of RBC from infected fish revealed inclusions containing reovirus like particles in the cytoplasm (Fig. 6A). The viral particles had an electron dense inner core with a diameter of X-Y nm surrounded by a less electron dense outer coat of X-Y nm. The viral particles lacked envelopes, and had a hexagonal outline (Fig. 6B). These features are consistent with members of the reovirus family.

The size of the inclusions varied from 100 to 1000 nm. Many inclusions were enclosed within membrane-like structure that contained numerous viral particles, whereas in others the membrane were partly broken or absent with more scattered virus particles (Fig. 6C, D). Some inclusions contained a lamellar structure with or without viral particles present (Fig. 6E). These differences may represent different stages of the viral replication cycle.

### Discussion

In this study it was found that RBC is a major target cell for PRV. High viral titers in RBC as well as viral factories containing reo-virus like particles, as found by RT-qPCR, flow cytometry and EM, respectively, are all observations supportive of the conclusion that PRV replicates in Atlantic salmon erythrocytes.

In Challenge # 1 we found that blood contained significantly higher viral loads than heart and skeletal muscle throughout the study period. In early phases there was significantly more virus in blood compared to all organs analyzed, including the spleen and head-kidney. The RT-qPCR results from Challenge # 2 revealed that a major part of the virus in blood was present in the erythrocyte fraction. This was corroborated by the results of flow cytometry where over 50% PRV positive RBC was observed in individual fish, and with the large majority of virus being detected intracellularly. The challenges were done at VESO Vikan, Namsos, Norway.

Using immunofluorescence and confocal microscopy it was detected that PRV was condensed into large globular structures localized to the cytoplasm of infected RBC. The flow cytometry analysis showed that highly PRV positive RBC had a significantly higher mean side scatter, thus more granular, compared to the PRV negative population in an infected individual. Orthoreovirus infected cells are associated with phase dense inclusions in the cytoplasm called viral factories. The viral factories contain large amounts of viral proteins, dsRNA (31) and partially or fully assembled particles, and are considered to be the location where viral RNA replication and packaging, and assembly of progeny particles take place. The inclusions observed in this study strongly resembled those described for orthoreoviruses, more specifically the globular viral factories seen in MRV type 3 prototype strain, Dearing (T3D), infected cells, in contrast to the microtubule-associated filamentous factories of the type 1 prototype strain, Lang (T1L) (15). The capacity of MRV to form viral factories facilitates successful viral replication.

The viral inclusions observed in this study were found by TEM to contain large amounts of reovirus-like particles. The virion lacked envelope, had a dense core of 30 nm and an outer capsid of 72 nm with a hexagonal outline. These features are in agreement with members of the Reoviridae family (16). The inclusions varied in size from 100 to 1000 and were often located in the perinuclear region. The content of the inclusions differed as some were filled with reovirus-like particles but others contained a mixture of virus particles and a more homogenous material with lamellar structures. The nature of these ladder structures could not be determined. MRV inclusions have been shown to contain both microtubules and intermediate filaments. It has also been described that the intermediate filament vimentin is rearranged in reovirus-infected CV-1 cells, both surrounding and incorporating into the viral inclusions. Most of the inclusions detected were encapsulated by a membrane like structure, in others the membrane seemed to be broken or absent. Mammalian orthoreovirus inclusions are not associated with membranes or other cellular organelles. Whether the membrane-like structure observed in this study was constituted of lipids or proteins was not determined.

Phylogenetic analysis have indicated that the PRV branches off the root of Orthoreovirus and the Aquareovirus genera (3). However, recent studies have concluded that PRV is more related to the Orthoreovirus genus and that it encodes a protein homologous to the mammalian orthoreoviruses (MRV) o1 cell attachment protein (4, 12). Interaction between viruses and erythrocytes is a characteristic feature shared by members of different virus families, including Reoviridae. MRV agglutinate erythrocytes via the o1 protein, a binding mediated by a sialic acid binding motif (17). This motif is partly conserved in PRV σ1 (4). In light of the phylogenetic relationship between MRV and PRV it is interesting to note that virus and erythrocyte interaction is conserved. PRV could serve as an interesting model in understanding the phylogenetic relationships in the family Reoviridae as it currently is the only known fish virus related to the Orthoreovirus genus.

Mammalian erythrocytes are devoted to oxygen transport and they undergo enucleation and lack organelles before entering circulation; an evolutionary trait that have benefited their oxygen-carrying capacity and also made them resistant to viral infections (). Cold water fish, like Atlantic salmon have lower oxygen demand and metabolism and thus less dependent on high oxygen transport. An extreme example of low oxygen demand is the antarctic hemoglobinless icefish (Channichthyidae) that lack erythrocytes (18). Most non-mammalian erythrocytes, including those of fish, are nucleated and contain organelles, and can therefore potentially serve as hosts for viral replication (19). In this study individual fish were shown to have over 50% PRV infected RBC as seen by flow cytometry. The sensitivity of the flow cytometry analysis only enabled detection of infected RBC in individuals with Ct-values of RT-qPCR below 20, thus the prevalence of PRV infected cells is probably significantly higher. Considering that RBC is the most abundant cell in blood, the total viral load in an infected fish would be substantial.

Studies have shown that the red blood cell population of fish in vivo is a mixture of cells of different ages (). Among the positive erythrocyte population detected by immunofluorescence microscopy there were variations in cell morphology, as some was identified as mature erythrocytes whereas others resembled immature erythrocytes. A mature red blood cells is characterized by the ellipsoidal shape with an elongated compact nucleus, opposed to the immature erythorytes (polychromatocytes) that tends to have a more circular cellular and nuclear shape (20). The erythrocyte maturation stages differ in cellular content as the amount of RNA and organelles decrease with increasing cellular age. Young red blood cells contain a higher total concentration of RNA and aging cells display a loss of cellular organelles including ribosomes and mitochondria (21). This could suggest that the more cellular active juvenile erythrocyte can better support viral replication. Although human mature erythrocytes cannot support a productive viral infection, Colorado tick fever virus (CTFV), a reovirus of the genus Orbivirus, have been detected in mature erythrocytes. This is thought to be the result of infected nucleated immature cells rather than of direct entry and replication of CTFV in mature erythrocytes, as CTFV has been shown to replicate in erythroblasts and reticulocytes of infected mice and in human hematopoietic progenitor cells. In farmed Atlantic salmon farming factors like reduced oxygen supply, due to high animal density, or increasing temperatures, spring and summer, stimulate erythropoiesis and would therefore be expected to increase the relative proportion of juvenile cells. The differences in red blood cell properties could have important implications for the PRV replication and outcome of the infection. The spherical shaped RBC observed in this study could also be a result of a stress induced red blood cell swelling (Sorensen), and not necessarily indicate immature erythrocytes. Future studies should investigate the replicative potential for PRV in hematopoietic cells. In addition, the question of other potential target cells in the blood remains to be answered.

There have been a number of reports of viral inclusions in salmonid erythrocytes that have collectively been termed erythrocytic inclusion body syndrome (EIBS) (11). No specific viruses have been assigned as the causative agent to these findings. Erythrocytic inclusions assumed to of viral origin have been observed in salmonid species in both Atlantic and Pacific coastlines like Atlantic salmon, rainbow trout (O. mykiss), chinook salmon (O. tshawythscha) and coho salmon (O.kisutch). The PRV particles and inclusions described in this study has a striking resemblance to several of the earlier reported viral inclusions described as EIBS (11, 22, 23). These observations all describe membrane bound viral inclusions containing a naked virus of the same size as reported in this study and are observed from both healthy and diseased fish, the latter with clinical signs including anemia and septicemia. The lamellar structure described here are present in many of these reports. The findings indicate that PRV is making up a part of the term EIBS, and that it should be called erythrocytic PRV inclusions when verified specifically.

Several studies have been conducted in order to reveal of the significance of PRV infection regarding the health of farmed Atlantic salmon. PRV has been shown to be the etiological cause of HSMI, as the cardiac viral load correlates with disease development and PRV antigen have been detected in cardiomyocytes coinciding with the course of the disease (8, 9). However, the present study shows that replication in red blood cells is an important feature of PRV infection that is not encompassed by the diagnosis of HSMI. Although HSMI is caused by PRV, the disease should be considered as a possible outcome of a PRV infection. The replication of PRV in erythrocytes could have broader implications for the fish health irrespective of the presence of heart lesions.

The diagnosis of HSMI is currently based on histopathological changes in the heart and skeletal muscle. In light of the high viral titers in blood and erythrocytes during an infection, it is interesting to note that it is the highly vascularized parts of these tissues that are usually most affected. In the heart, the myocardial lesions are initially observed in the outer compact layer and epicardium that, in contrast to the inner spongy layer, contains many blood vessels branching off from the coronary arteries. The early histopathological lesions extend from the epicardium and into the compact layer particularly along small vascular structures. This could tentatively be attributed to the close exposure to virus in the blood during an infection. In the skeletal muscles, lesions are located to the red skeletal muscle. This muscle layer is also highly vascularized (55), compared to the white skeletal muscle which appears unaffected during HSMI.

As opposed to the heart and skeletal muscle, lesions in the spleen and head kidney are not included in the diagnostic criteria of HSMI, however, the latter organs contain more virus (24); a finding that was confirmed in this study. A part of these high viral titers could be assigned to residual PRV infected RBC. In addition, both organs are immunologically important in the clearance of viral infections, thus influencing the PCR readings. It should not be ruled out that PRV can replicate in non-RBC cells in the head-kidney or spleen. It is also interesting to note that the hematopoietic tissue in Atlantic salmon is primarily located to the head kidney and spleen.

PRV is found to be ubiquitously distributed in healthy farmed Atlantic salmon in Norway, Canada and Chile, as well as found with less prevalence in wild Atlantic salmon and brown trout (*Salmo trutta L.)* in Norway, and wild Chum salmon (Oncorhynchus keta), Rainbow trout (*O*. *mykiss*) and Cutthrout trout (*O*. *clarkii*) in Canada (6, 7). The widespread occurrence of PRV in healthy farmed and wild fish could reflect replication in erythrocytes at a level that do not induce the heart and skeletal muscle lesions seen in HSMI outbreaks. Field observations indicate that PRV is present in populations of farmed Atlantic salmon long after cessation of an HSMI outbreak. Persistent infection of erythrocytes could explain these findings, as fish erythrocytes are thought to be in circulation for as long as 6 months (21). It is important to note that the presence of PRV in circulating erythrocytes will influence the amount of virus detected by qPCR screening of any organ including heart, skeletal muscle, head-kidney and spleen. Residual blood containing PRV positive cells would affect the qPCR results obtained even if resident cells are not infected.

In conclusion, the replication of PRV in erythrocytes provides new information about the HSMI pathogenesis, and defines PRV as an important factor of viral erythrocytic inclusions.

**Table 1. PRV Ct-values from RBC in Challenge #2.**

| **Wpc** (Fish ID) | **PRV Ct-value (RBC)** | | | | | |
|---|---|---|---|---|---|---|
| **0 wpc** (F01-06) | 35,0 | 35,0 | 35,0 | 35,0 | 35,0 | 35,0 |
| **3 wpc** (F31-36) | 35,0 | 35,0 | 35,0 | 35,0 | 35,0 | 35,0 |
| **4 wpc** (F41-46) | 35,0 | 35,0 | 35,0 | 35,0 | 35,0 | 26,9 |
| **5 wpc** (F51-56) | 28,2 | 25,1 | 29,1 | 27,1 | 19,0 | 19,3 |
| **6 wpc** (F61-66) | 17,8 | 23,9 | 16,8 | 16,1 | 16,8 | 19,5 |
| **7 wpc** (F71-76) | 16,8 | 15,5 | 15,9 | 23,2 | 17,5 | 16,0 |
| **8 wpc** (F81-86) | 15,7 | 17,5 | 16,5 | 18,1 | 17,9 | 17,8 |

| **Wpc** | | **Ct-values** | | | | | | **Mean (±SD)** |
|---|---|---|---|---|---|---|---|---|
| | | *F01* | *F02* | *F03* | *F04* | *F05* | *F06* | |
| | Blood | 35,0 | 35,0 | 35,0 | 35,0 | 35,0 | 35,0 | 35,0 (±0,0) |
| | Heart | 35,0 | 35,0 | 35,0 | 35,0 | 35,0 | 35,0 | 35,0 (±0,0) |
| **0** | SM | 35,0 | 35,0 | 35,0 | 35,0 | 35,0 | 35,0 | 35,0 (±0,0) |
| | Spleen | 35,0 | 35,0 | 35,0 | 35,0 | 35,0 | 35,0 | 35,0 (±0,0) |
| | HK | 35,0 | 35,0 | 35,0 | 35,0 | 35,0 | 35,0 | 35,0 (±0,0) |

| | | *F41* | *F42* | *F43* | | | | |
|---|---|---|---|---|---|---|---|---|
| | Blood | 35,0 | 35,0 | 35,0 | | | | 35,0 (±0,0) |
| | Heart | 35,0 | 35,0 | 35,0 | | | | 35,0 (±0,0) |
| **4** | SM | 35,0 | 35,0 | 35,0 | | | | 35,0 (±0,0) |
| | Spleen | 35,0 | 35,0 | 35,0 | | | | 35,0 (±0,0) |
| | HK | 35,0 | 35,0 | 35,0 | | | | 35,0 (±0,0) |

| | | *F61* | *F62* | *F63* | *F64* | *F65* | *F66* | |
|---|---|---|---|---|---|---|---|---|
| | Blood | Na | 23,6 | 35,0 | 29,4 | 24,2 | 27,3 | 27,9 (±4,6) |
| | Heart | 31,5 | 30,5 | 35,0 | 34,4 | 30,7 | 35,0 | 32,9 (±2,2) |
| **6** | SM | 35,0 | 35,0 | 35,0 | 35,0 | 35,0 | 35,0 | 35,0 (±0,0) |
| | Spleen | 30,4 | 27,7 | 35,0 | 32,2 | 27,2 | 31,0 | 30,6 (±2,9) |
| | HK | 31,2 | 30,9 | 35,0 | 33,0 | 29,3 | 33,8 | 32,2 (±2,1) |

| | | *F81* | *F82* | *F83* | *F84* | *F85* | *F86* | |
|---|---|---|---|---|---|---|---|---|
| | Blood | 18,3 | 17,0 | 18,9 | 18,7 | 18,0 | 17,6 | 18,1 (±0,7) |
| | Heart | 23,3 | 22,4 | 21,6 | 24,2 | 20,7 | 22,3 | 22,4 (±1,2) |
| **8** | SM | 27,1 | 27,7 | 27,7 | 26,9 | 26,5 | 27,1 | 27,2 (±0,5) |
| | Spleen | 17,5 | 17,2 | 16,5 | 20,0 | 14,9 | 16,2 | 17,1 (±1,7) |
| | HK | 18,8 | 18,2 | 16,8 | 20,7 | 17,4 | 18,4 | 18,4 (±1,4) |

| | | *F101* | *F102* | *F103* | *F104* | *F105* | *F106* | |
|---|---|---|---|---|---|---|---|---|
| | Blood | 22,4 | 18,9 | 17,0 | 18,6 | 21,3 | 17,7 | 19,3 (±2,1) |
| | Heart | 22,9 | 22,3 | 17,4 | 23,5 | 27,3 | 22,6 | 22,7 (±3,2) |
| **10** | SM | 32,9 | 29,2 | 27,0 | 29,4 | 32,7 | 28,1 | 29,9 (±2,4) |
| | Spleen | 16,7 | 16,2 | 15,5 | 17,0 | 21,6 | 16,1 | 17,2 (±2,2) |
| | HK | 18,6 | 18,8 | 17,1 | 18,6 | 22,9 | 18,3 | 19,0 (±2,0) |

| | | *F121* | *F122* | *F123* | *F124* | *F125* | *F126* | |
|---|---|---|---|---|---|---|---|---|
| | Blood | 20,1 | 21,7 | 23,3 | 27,4 | 35,0 | 22,1 | 24,9 (±5,5) |
| | Heart | 25,7 | 26,6 | 29,4 | 28,7 | 35,0 | 27,6 | 28,8 (±3,3) |
| **12** | SM | 28,1 | 29,0 | 32,0 | 32,5 | 35,0 | 28,6 | 30,8 (±2,6) |
| | Spleen | 17,9 | 18,7 | 20,5 | 20,3 | 35,0 | 18,3 | 21,8 (±6,6) |
| | HK | 20,3 | 18,6 | 20,0 | 21,8 | 35,0 | 20,3 | 22,7 (±6,1) |

| | | *F141* | *F142* | *F143* | *F144* | *F145* | *F146* | |
|---|---|---|---|---|---|---|---|---|
| | Blood | 22,7 | 19,9 | 20,3 | 22,7 | 23,9 | 23,2 | 22,1 (±1,6) |
| | Heart | 28,4 | 25,9 | 25,7 | 27,9 | 29,4 | 27,7 | 27,5 (±1,5) |
| **14** | SM | 33,4 | 30,7 | 29,4 | 33,3 | 32,1 | 30,1 | 31,5 (±1,7) |
| | Spleen | 21,4 | 19,5 | 16,8 | 19,1 | 19,5 | 17,2 | 18,9 (±1,7) |
| | HK | 21,5 | 21,6 | 19,1 | 21,5 | 22,1 | 19,8 | 20,9 (±1,2) |

Table S1. Piscine orthoreovirus (PRV) Ct-values in blood, heart, skeletal muscle (SM), spleen and head-kidney (HK) from challenge experiment 1 sampled at 0, 4, 6, 8, 10, 12 and 14 weeks post challenge (Wpc). The mean Ct-value and standard deviation (Mean (±SD)) for the different tissues are shown in the right column. A unique identifier for the fish sampled are included (Ex. F101-F106). Na, not available; Ct, cycle threshold.

**Table S2**

| **Wpc** | | **PRV Ct-values** | | | | | | **Mean (±SD)** |
|---|---|---|---|---|---|---|---|---|
| | | *F01* | *F02* | *F03* | *F04* | *F05* | *F06* | |
| | Blood | 35,0 | 35,0 | 35,0 | 35,0 | 35,0 | 35,0 | 35,0 (±0,0) |
| **0** | RBC | 35,0 | 35,0 | 35,0 | 35,0 | 35,0 | 35,0 | 35,0 (±0,0) |
| | Spleen | 35,0 | 35,0 | 35,0 | 35,0 | 35,0 | 35,0 | 35,0 (±0,0) |
| | Plasma | 35,0 | 35,0 | 35,0 | 35,0 | 35,0 | 35,0 | 35,0 (±0,0) |

| | | *F31* | *F32* | *F33* | *F34* | *F35* | *F36* | |
|---|---|---|---|---|---|---|---|---|
| | Blood | 35,0 | 35,0 | 35,0 | 35,0 | 35,0 | 35,0 | 35,0 (±0,0) |
| **3** | RBC | 35,0 | 35,0 | 35,0 | 35,0 | 35,0 | 35,0 | 35,0 (±0,0) |
| | Spleen | 35,0 | 35,0 | 35,0 | 35,0 | 35,0 | 35,0 | 35,0 (±0,0) |
| | Plasma | 35,0 | 35,0 | 35,0 | 35,0 | 35,0 | 35,0 | 35,0 (±0,0) |

| | | *F41* | *F42* | *F43* | *F44* | *F45* | *F46* | |
|---|---|---|---|---|---|---|---|---|
| | Blood | 35,0 | 35,0 | 35,0 | 34,5 | 34,8 | 24,4 | 33,1 (±4,3) |
| **4** | RBC | 35,0 | 35,0 | 35,0 | 35,0 | 35,0 | 26,9 | 33,7 (±3,3) |
| | Spleen | 35,0 | 35,0 | 35,0 | 35,0 | 35,0 | 28,4 | 33,9 (±2,7) |
| | Plasma | 35,0 | 35,0 | 35,0 | 35,0 | 35,0 | 26,7 | 33,6 (±3,4) |

| | | *F51* | *F52* | *F53* | *F54* | *F55* | *F56* | |
|---|---|---|---|---|---|---|---|---|
| **5** | Blood | 30,2 | 22,0 | 27,0 | 25,2 | 16,9 | 16,9 | 23,0 (±5,4) |
| | RBC | 28,2 | 25,1 | 29,1 | 27,1 | 19,0 | 19,3 | 24,6 (±4,4) |
| | Spleen | 32,9 | 26,4 | 29,3 | 27,1 | 19,6 | 19,3 | 25,8 (±5,4) |
| | Plasma | 37,3 | 23.5 | 28,5 | 25,8 | 18,5 | 17,9 | 25,2 (±7,2) |

| | | *F61* | *F62* | *F63* | *F64* | *F65* | *F66* | |
|---|---|---|---|---|---|---|---|---|
| **6** | Blood | 14,9 | 21,6 | 14,4 | 15,8 | 15,5 | 16,7 | 16,5 (±2,6) |
| | RBC | 17,8 | 23,9 | 16,8 | 16,1 | 16,8 | 19,5 | 18,5 (±2,9) |
| | Spleen | 18,0 | 25,8 | 17,7 | 18,0 | 17,0 | 19,0 | 19,2 (±3,3) |
| | Plasma | 17,9 | 25,2 | 16,1 | 15,8 | 15,5 | 18,6 | 18,2 (±3,6) |

| | | *F71* | *F72* | *F73* | *F74* | *F75* | *F76* | |
|---|---|---|---|---|---|---|---|---|
| **7** | Blood | 17,7 | 14,7 | 16,1 | 20,3 | 14,2 | 17,1 | 16,7 (±2,2) |
| | RBC | 16,8 | 15,5 | 15,9 | 23,2 | 17,5 | 16,0 | 17,5 (±2,9) |
| | Spleen | 18,9 | 17,9 | 17,9 | 22,2 | 17,6 | 17,8 | 18,7 (±1,7) |
| | Plasma | 24,4 | 23,5 | 22,6 | 22,2 | 13,3 | 23,3 | 21,5 (±4,1) |

| | | *F81* | *F82* | *F83* | *F84* | *F85* | *F86* | |
|---|---|---|---|---|---|---|---|---|
| | Blood | 16,4 | 17,2 | 16,8 | 19,4 | 18,3 | 17,2 | 17,6 (±1,1) |
| **8** | RBC | 15,7 | 17,5 | 16,5 | 18,1 | 17,9 | 17,8 | 17,2 (±0,9) |
| | Spleen | 15,8 | 16,8 | 16,7 | 16,9 | 16.1 | 17,0 | 16,5 (±0,5) |
| | Plasma | 22,4 | 22,8 | 21,6 | 24,2 | 24,4 | 23,6 | 23,2 (±1,1) |

Table S2. Piscine orthoreovirus (PRV) Ct-values in blood, RBC, spleen and plasma from challenge experiment 2 sampled at 0 and 3-8 weeks post challenge (Wpc). The mean Ct-value and standard deviation (Mean (±SD)) for the different material are shown in the right column. A unique identifier for the fish sampled are included (Ex. F01-F06).

### Example 2

### Ex vivo culture of erythrocytes

Erythrocytes were isolated from adult Atlantic salmon (approx. 2 kg, NIVA ,Solbergstrand, Norway), which was confirmed uninfected with PRV by RT-qPCR (spleen). Cells were washed, counted and dissolved to 5 mill/ml in Leibovitz L15 media supplemented with fetal calf serum (10%) and gentamicin (50 µg/ml). Cells were plated in a 24 well plate with 5 mill cells/well and kept on gentle agitation at 15°C in a non-CO₂ incubator. The next day, freshly isolated erythrocytes from three separate fish infected with PRV (Challenge experient # 2, cohabitants, 6WPC) was added (0.1 mill cells/well), and cells were incubated for up to 13 days (15°C). 250 µl cells were harvested per time-point in sterile eppendorff tubes and centrifuged (750 x G, 5 min). The media was discarded, and RNA was isolated from the erythrocyte pellet.

### Results

A pilot experiment was performed to see if PRV can be propagated in red blood cells in culture *ex vivo,* and obtained results showing a 120-fold increase in two weeks. The RBC viability in culture was excellent for the 2 weeks tested. Figure 8 shows the mean increase in viral counts (RT-qPCR absolute quantification) 6 and 13 days after introduction of virus.

### Example 3

### Materials and methods

**Blood from *in vivo* PRV infected fish.** A PRV challenge experiment using ten unvaccinated, seawater-adapted Atlantic salmon with an initial weight of 100 g was conducted at VESO Vikan aquatic research facility, Vikan, Norway. The fish originated from a naive population at the research facility confirmed free of PRV, ISAV, salmonid alphavirus (SAV), piscine myocarditis virus (PMCV) and infectious pancreatic necrosis virus (IPNV) by reverse transcription quantitative PCR (RT-qPCR). Currently, viral haemorrhagic necrosis virus (VHSV) is not present in Norwegian fish farming, and was not screened for. The fish were acclimatized for 2 weeks prior to challenge and kept in tanks supplied with filtered and UV-radiated seawater with 32-33 ‰ salinity at 12°C, and with a regime of 24 h light / 0 h darkness. Before sampling the fish were anesthetized by bath immersion (2-5 min) in benzocaine chloride (0.5 g/10 L water) (Apotekproduksjon AS; Oslo, Norway), and euthanized after sampling using benzocaine chloride (1 g/5 L water) for 5 min. The challenge experiment had been approved by the Norwegian Animal Research Authority.

A blood pellet prepared in a previous challenge experiment (see Example 1) containing high loads of PRV, as measured by RT-qPCR (Ct-value 19.9), were used as challenge material. The material had been confirmed free of ISAV, IPNV, SAV and PMCV. The blood pellet was thawed, diluted 1:1 in phosphate-buffered saline (PBS), and administered by intraperitoneal (i.p.) injection using 0.1 mL inoculum per fish. Two fish, i1 and i2, were sampled 5 weeks post challenge (wpc) collecting peripheral blood from the caudal vein into heparinized vacutainers.

**Blood from naive fish.** Five naive seawater-adapted Atlantic salmon, n1-n3, with a weight of 226 - 374 g were sampled at Solbergstrand marine research station, Drøbak, Norway. The fish had been vaccinated one year earlier by i.p. injection using a vaccine containing bacterial proteins with no adjuvant and were kept in a seawater tank using a regime of 12 h light / 12 h darkness. Prior to sampling, the fish were anesthetized by bath immersion (2-5 min) in benzocaine chloride (0.5 g/10 L water) and euthanized after sampling by a blow to the head. Sampled peripheral blood into heparinized vacutainers from all three naïve fish (n1-n3).

**Isolation of RBC.** RBC were isolated from the heparinized blood collected from both naïve (n1 - n3) and *in vivo* infected fish (i1, i2) using a previously described Percoll gradient (Example 1). Briefly, blood was diluted 1:10 in distilled PBS (dPBS), layered onto a discontinuous Percoll gradient of 34% - 49% (GE Healthcare, Uppsala, Sweden), centrifuged at 2000 x g for 20 min at 4 °C before RBC were collected at the bottom of the gradient. The 34 % - 49 % interface, containing residual erythrocytes, was re-layered onto a fresh gradient and centrifuged at 500 x g for 10 min at 4 °C. The RBC from the bottom of this gradient were collected, pooled with the erythrocytes from the first gradient, and washed twice in PBS. The cells were counted using Countess (Invitrogen, Eugene, Oregon, USA) and resuspended in Leibovitz's L15 (Life Technologies, Carlsbad, CA, USA) medium supplemented with fetal calf serum (10%) and gentamicin (50 µg/ml).

**PRV *ex vivo* infection experiment.** Isolated RBC from *in vivo* infected fish i1 and i2 were confirmed PRV-positive by RT-PCR (described below). Batches of i1 and i2 RBCs of 1 x 10⁷ cells / mL were freeze-thawed once at -80 °C and centrifuged at 800 g for 5 min at 4 °C. The supernatant was collected and used as inoculum in passage of PRV.

RBC from naïve fish n1, n2 and n3 were confirmed PRV-negative by RT-PCR (described below) and used as naive recipient RBC in passage of PRV. In 12-well plates, 2 mL of RBC in L15 medium (1 x 10⁷ cells/mL) were added per well. The plate was incubated with gentle agitation at 12 °C overnight. The following day, 100 µL RBC inoculum from *in vivo* PRV infected fish i1 or i2 were added to the wells and incubated with gentle agitation at 12°C until sampled. The plates were harvested at 1, 6, 12 and 19 dpi (one plate per sampling). One millilitre, i.e. 1 x 10⁷ RBC, was transferred to sterile eppendorf tubes and centrifuged at 750 g for 5 min, and the supernatant was put in separate tubes. Total RNA was isolated from the erythrocyte pellet for qPCR analysis. The remaining 1 x 10⁷ RBC, was used for flow cytometry analysis, immunofluorescence and preparation of blood smears. All analysis is described below.

**Second passage.** Material from a plate treated like in the first passage was used as inoculum in the second passage. At day 22 dpi, two of the infected wells were chosen as inoculum: naïve fish n2 infected with i1 (n2-i1) and naïve fish n2 infected with i2 (n2-i2). Batches of 1 x 10⁷ cells / mL were freeze thawed once at -80 °C and centrifuged at 800 g for 5 min at 4 °C and 10 fold dilutions of the supernatant was used as inoculums. RBC from naïve fish n4 and n5 were used as recipients in the second passage, and inoculated with 100 µL of 10°, 10⁻¹ and 10⁻² dilutions of the n2-i1 and n2-i2 preparations. At 1 dpi and 15 dpi the plates were sampled for RT-qPCR analysis.

**RNA isolation.** Total RNA was isolated from 1 x 10⁷ RBC as previously described (Example 1). Briefly, RBC was homogenized in QIAzol Lysis Reagent (Qiagen, Hilden, Germany), chloroform was added, the phases were separated, and RNA extracted from the aqueous phase using RNeasy Mini spin column (Qiagen), following the manufacturer's instructions. RNA was quantified using a NanoDrop ND-1000 spectrophotometer (Thermo Fisher Scientific, Wilmington, DE, USA).

**qPCR.** Reverse transcription was performed using 200 ng total RNA per sample, and a mixed input from 12 representative samples was used to prepare a seven point concentration standard curve. The RNA was denaturated at 95 °C for 5 min prior to cDNA synthesis, which was performed using the Quantitect reverse transcription kit with integrated genomic DNA removal (Qiagen). Quantitative PCR (qPCR) was run in duplicate on cDNA corresponding to 10 ng RNA input. The PRV analysis was performed using Quantifast probe qPCR Master Mix (Qiagen) and 400 nM specific primers and 200 nM probe targeting the S1 segment encoding the PRV σ3 gene. The amplification was run for 40 cycles of 94 °C/15 s, 54°C/30 s, 72°C/30 s in a Mx3005P device (Stratagene, La Jolla, CA, USA). An absolute quantification standard curve of cDNA made from in vitro transcripts of the σ3 gene (10⁷ to 10 transcripts) was run on the same plate and used to calculate counts of target viral RNA molecules. Primers and probe used are listed in Table 2.

For antiviral response gene expression analysis, the master mix and primer/probe concentrations given above were used for the targeting of A. salmon IFNα, Mx and RIG-I. The assays were run 40 cycles of 94 °C/15 s, 60°C/30 s. Levels of elongation factor 1 α (EF1α) mRNA were assessed using 500 nM primers and the Maxima SYBR Green/ROX qPCR Master Mix (Fisher scientific) with the same cycle conditions. Primers and probe used are listed in Table 2. The specificity of the SYBR Green assay was confirmed by melting point analysis. The concentration standard curve was run on each plate and used to calculate relative differences. Levels of EF1α mRNA were used for normalization.

**Table 2. Primers used in Example 2. Quenchers and reported dye of the probes in italic.**

| **Primer/probes** | **Primer sequence (5'** → **3')** |
|---|---|
| o'3 S1-659 Fwd | TGCGTCCTGCGTATGGCACC (SEQ ID NO:1) |
| o'3 S1-801 Rev | GGCTGGCATGCCCGAATAGCA (SEQ ID NO:2) |
| o'3 S1-693 Probe | *FAM*-ATCACAACGCCTACCT-*MGBNFQ* (*FAM*-SEQ ID NO:3-*MGBNFQ*) |
| IFNα Fwd | ACTGAAACGCTACTTCAAGAAGTTGA (SEQ ID NO:4) |
| IFNα Rev | GCAGATGACGTTTTGTCTCTTTCCT (SEQ ID NO:5) |
| IFNα Probe | *FAM*-TTGATGGGCTGGATGACTTTAGGA-*TAMRA* (*FAM*-SEQ ID NO:6-*TAMRA*) |
| Mx Fwd | GATGCTGCACCTCAAGTCCTATTA (SEQ ID NO:7) |
| Mx Rev | CACCAGGTAGCGGATCACCAT (SEQ ID NO:8) |
| Mx Probe | *FAM*-CTGATCAGCCAAACGTTGACTGGATATCCT-*TAMRA* (*FAM*-SEQ ID NO:9-*TAMRA*) |
| RIG-I Fwd | ACGCCTTGAAGAGCTGGATA (SEQ ID NO:10) |
| RIG-I Rev | CTGGCTGGACTTGTGTCCTC (SEQ ID NO:11) |
| RIG-I Probe | *FAM*-CCTGAAACCAGGAGTACTGATCGGGA-*TAMRA* (*FAM*-SEQ ID NO:12-*TAMRA*) |
| EF1α Fwd | TGCCCCTCCAGGATGTCTAC (SEQ ID NO:13) |
| EF1α Rev | CACGGCCCACAGGTACTG (SEQ ID NO:14) |

**Data analysis.** The PRV q-PCR results, from 1 dpi of the *ex vivo* infection experiment were compared to paired samples collected at 6, 12 and 19 dpi. Similar comparison was performed for IFNα, Mx and RIG-I, assigning the qPCR results from uninfected naive fish as 1 and calculating the fold increase at 1, 6, 12 and 19 dpi. The differences were analysed statistically using Wilcoxon matched pairs signed rank test due to the sample size (n=6).

**Flow cytometry.** RBC sampled during the *ex vivo* infection experiment (1, 6, 12 and 19 dpi), in addition to RBC isolated from *in vivo* infected with PRV (i1, i2) and the naïve recipient RBC (n1-n3) were screened for PRV by flow cytometry. The RBC were stained intracellularly using a polyclonal antibody raised in rabbits against putative PRV outer capsid protein o1 (Anti-o'1, #K275) as previously described (Example 1). Briefly, RBC were plated into 96-well plates at densities of 1.0 x10⁶ cells per well, washed in staining buffer (PBS + 1 % BSA + 0.05 % azide), fixed and permeabilized by incubation in Cytofix/Cytoperm (Becton Dickinson, San Diego, CA, USA) and stained using primary antibody Anti-σ1 (1:10 000) and secondary Alexa Fluor 488 conjugated anti-rabbit IgG (Molecular Probes, Eugene, Oregon, USA) (2mg/mL diluted 1:800). The corresponding zero serum (Anti-σ1 Zero #K275) [8] was used as negative control serum. The cells were read on a Gallios Flow Cytometer (Beckman Coulter, Miami, FL, USA) counting 50 000 cells per sample. The data were analyzed using the Kaluza software (Becton Dickinson).

**Immunofluorescence microscopy.** The PRV stained RBC used in flow cytometry were also inspected by immunofluorescence microscopy. The nuclei were stained with propidium iodide (0.5 µg/mL, Molecular Probes) before they were transferred to Countess chamber slides (Invitrogen). Photographs were taken by an inverted fluorescence microscope (Olympus IX81), at 20x and 40x magnification.

**Monitoring the *in vivo* infected RBC in culture.** Separate from the *ex vivo* infection experiment described above, the remaining RBC from *in vivo* infected fish i1 and i2, already infected with PRV, were kept in culture for 19 days to study the cell morphology and inclusions of PRV infected RBC, using naïve RBC n1-n3 as controls. These were sampled at 1, 12 and 19 days in culture and studied as described below.

**Confocal microscopy.** Smears of RBC of *in vivo* infected i1 and i2 kept in culture were prepared, and cells were dried before fixation in ice cold methanol. Slides were rehydrated in PBS and blocked in PBS with 5% skimmed milk before sequentially staining with monoclonal antibody against dsRNA (J2; 1:200) (Scicons, Hungary) and rabbit anti-sigma1 (1:500) with washing in-between. Secondary antibody; goat antimouse Alexa Fluor 488 and goat anti-rabbit Alexa Fluor 647 (1:1000) were obtained from Molecular Probes (Invitrogen). Nucleus was counterstained with propidium iodide (0.5 µg/ml) and coverslips were mounted with Fluoroshield (Sigma-Aldrich). Non-saturated images were captured with sequential scanning by a Plan-Apochromat 63/1.4 oil objective in a Zeiss laser scanning confocal microscope (Zeiss Axiovert 200M fluorescent inverted microscope, equipped with a LSM 510 laser confocal unit, 488 nm argon laser, 546 and 633 nm helium/neon laser (Carl Zeiss, Jena, Germany)).

**Pinacyanol chloride staining.** RBC smears, both from the *ex vivo* infection experiment and *in vivo* infected i1 and i2, were stained with pinacyanol chloride as described previously, with minor modifications (56) . Briefly, cells were fixed for 5 min in absolute methanol, and stained for 1 minute with filtered pinacyanol chloride solution (0.25 g pinacyanol chloride dissolved in 36.7 ml 95% Ethanol and 13.25 ml distilled water). Slides where then extensively rinsed in tap water before drying and mounting with fluoroshield (Sigma-Aldrich) right before examining in the microscope (Olympus IX-81 at 60x magnification). Images were captured with a color camera and later converted to grayscale in Adobe Photoshop Elements to better contrast the inclusions.

### Results

**Naive and infected RBC.** RBC infected by PRV *in vivo* (i1, i2) and the recipient naïve RBC (n1-n3) were screened for PRV by qPCR and flow cytometry in preparation for the *ex vivo* infection experiment. The i1 and i2 both contained approximately 12 000 copies of the viral target sequence per 10 ng cDNA as measured by qPCR. All naïve erythrocytes (n1-n3) were confirmed as PRV negative by qPCR. When analyzing the *in vivo* infected i1 RBC by flow cytometry, 46% were detected as PRV infected, observed as a single low PRV positive population (Fig. 9A). In *in vivo* infected i2, 49 % of RBC were PRV positive, however, both a low PRV positive population and a smaller high positive population was observed by flow cytometry (Fig.9 A). All naive erythrocytes (n1-n3) were confirmed as PRV negative by flow cytometry before initiation of the *ex vivo* study (Fig.9A).

***Ex vivo* infection of RBC.** Erythrocytic lysate prepared from PRV infected i1 and i2 were passaged to naïve primary erythrocytes (n1-n3). During 19 days of *ex vivo* cultivation an increased PRV load was observed in all six cultures as measured by q-PCR. The mean viral loads at 12 and 19 dpi were significantly higher compared to 1 dpi (p<0.05) (Fig.9B). The three *ex vivo* infected naive RBC populations (n1-n3) produced more virus when infected with i2 erythrocytic lysate compared to i1 lysate (Fig.9C).

**PRV positive cells in *ex vivo* experiment by immunofluorescence.** During the *ex vivo* infection experiment the RBC samples were stained for PRV and analyzed by both flow cytometry and visually inspected by immunofluorescence (IF) microscopy. At 1 and 6 dpi no positive cells were observed by IF microscopy or by flow cytometry (Fig.10A). At 12 dpi single PRV positive RBC were observed by IF microscopy in all six samples (Fig.10B). The staining was observed as granular inclusions in the cytoplasm. At 19 dpi a few single PRV positive erythrocytes were observed in all samples. Again, the staining was detected as cytoplasmic inclusions, however more often as one prominent large inclusion (Fig.10C). The inclusions were visible in phase contrast both at 12 and 19 dpi, and co-localized with immunofluorescent staining of PRV (Fig.10B,C). PRV positive RBC tended to have a rounded cell shape and nucleus. Although a few PRV positive RBC were detected in all samples from 12 and 19 dpi, a positive population could not be observed by flow cytometry.

**RBC elicits an innate immune response.** In order to determine if the PRV infected RBC elicited an antiviral response to PRV infection, the gene expression levels of IFNα, the Myxovirus resistance gene (Mx) and retinoic acid-inducible gene (RIG)-I was analyzed along the course of the *ex vivo* infection experiment. IFNα, which is an early antiviral response gene, was upregulated one and six days after infection and declined at 12 dpi (Fig. 11A). The levels of Mx, which is an IFN-regulated gene, increased significantly (mean increase 15-fold) six days after PRV infection, followed by a slight decrease at 12 and 19 dpi (Fig. 11B). For the dsRNA helicase RIG-I, regulated in response to cytoplasmic dsRNA by an IFN-independent mechanism, a significant 8-fold increase was observed at 6 dpi and the expression stayed elevated throughout the experimental period (Fig. 11C). Individual naive RBC populations (n1-n3) showed somewhat different levels and timing of responses, but the same general trend (data not shown).

**Characterization of PRV inclusions and RBC morphology using *in vivo* infected RBC.** Separate from the PRV *ex vivo* infection experiment described above, remaining RBC from *in vivo* infected i1 and i2, were kept in culture and used in an observational study for 19 days to describe the cell morphology and inclusions of PRV infected RBC. RBC from naïve fish n1-n3 was used as controls. The RBC from naive fish were a homogenous population with similar shape and size typical for mature RBC. The RBC fraction from i1 and i2 were heterogenous, and both the nuclear and cell morphology of the RBC varied. In addition some smaller round cells were observed in the infected cultures.

Stained smears of i1 and i2 RBC showed that the inclusions contained o1 protein and dsRNA. When observed after 1 day in culture, RBC with small punctual staining of dsRNA and o1 in the cytoplasm (Fig. 12A), and RBC with more pronounced staining of o1 and dsRNA was detected (Fig. 12B). These latter cells appeared smaller than the ellipsoid RBC. In some of the characteristic ellipsoid RBC, only dsRNA staining were detected. This could indicate that the anti-dsRNA antibody is more sensitive to detect viral infection compared to anti-o'1. In general, PRV positive RBC of i2 had stronger and more frequent staining than those observed in i1. The controls (n1-n3) were negative for both dsRNA and o1 staining.

After 12 days in culture, RBC which stained strongly positive for dsRNA and o1 appeared shrunken with a fainter PI staining compared to PRV negative RBC (Fig.12C), indicating reduced viability. The cellular and nuclear shape of PRV infected RBC were typically rounder compared to negative cells. The inclusions observed day 12 were larger compared to the small punctual staining observed at day 1. The number of inclusions increased and some big inclusions positive for o'1 were observed. At day 19 dsRNA signals were observed more frequently than at earlier time points, and o1 inclusions were larger compared to day 1 (Fig. 12D). In addition, several apparently healthy ellipsoidal RBC were positive for dsRNA and o1 observed as small punctual staining (Fig. 12E). In general, cells with strong PI signal gave some unspecific signal in the nucleus when detecting o1 using Alexa 647 as fluorochrome, despite using a band pass filter of 635 nm. The dsRNA and o1 signal was sometimes co-localized but appeared also as separate staining.

**EIBS staining technique.** Inclusions stained positive by pinacyanol chloride staining have previously been observed in RBC from fish diagnosed with EIBS. Similar staining were frequently detected in both *in vivo* infected i1 and i2 in the present study, observed as small and large cytoplasmic inclusions (Fig. 13B, Bi and Bii). These inclusions were not observed in RBC from naïve fish (Fig. 13A). A reduction in the number of cytoplasmic inclusions were observed after 12 days in culture (Fig. 13C), and after 19 days inclusions appeared as separate big or small inclusions (Fig 13D, and inset) but also organized in clusters (Fig 13D, inset).

In the *ex vivo* infection experiment, using naive RBC infected with cell supernatant from *in vivo* infected RBC, cytoplasmic inclusions close to the nucleus could be observed at 12 dpi (Fig. 13E and inset), and at 19 dpi both inclusions and big cell "ghosts" were observed (Fig. 13F).

### Discussion

In this study it was demonstrated that PRV obtained from RBC of *in vivo* infected fish can be passaged to a culture of naive Atlantic salmon erythrocytes, and replicate *ex vivo.* This provides evidence that isolated RBC from infected fish contains infectious PRV particles, and that supernatant from lysed RBC can be used to propagate PRV. The erythrocytic PRV inclusions were shown to contain both PRV protein and dsRNA, and stained positive with pinacyanol chlorid. An innate antiviral immune response was generated in infected RBC.

The lack of cell lines supporting PRV replication has restricted studies of PRV pathogenesis. The present study demonstrated an *ex vivo* infection model using lysate from *in vivo* PRV infected RBC and primary naive erythrocytes, which could be used to produce a significant increase in viral loads 12 days after infection. In this model, a difference between RBC extracts from two different infected fish, i1 and i2, was observed as higher viral loads were obtained in naïve RBC infected with i2 erythrocyte lysate compared to i1. By flow cytometry it was shown that although the total cell numbers detected as PRV positive in the two cell populations i1 and i2 were similar (46% and 49 % respectively), the i2 RBC, contained a high PRV positive cell population in addition to the low positive population. Both low and high PRV positive populations have been described in PRV infected RBC during an *in vivo* experimental trial (Example 1), and the presence of a high positive population was associated with the peak phase of infection. This could indicate that the high positive population contains more infectious viral particles.

Using a polyclonal antibody directed against the PRV protein o1, PRV was detected in a few single cells by IF staining in the *ex vivo* infection experiment. However, none of the samples were detected as positive by flow cytometry (data not shown). In previously performed *in vivo* experimental trials, it was demonstrated that blood containing high PRV loads as measured by qPCR, could be used for flow cytometric counting of PRV infected cells (Example 1). However, the sensitivity of the Anti-o'1 antibody used for flow cytometry were too low to detect PRV positive RBC populations when the sample qPCR Ct-values were above 20. There are reasons to believe that the viral loads obtained in this study did not meet the threshold for detection by this method. The total number of PRV positive cells, or the amount of virus per cell, was probably too low to be observed as a distinct positive population in flow cytometry.

The presence of dsRNA and PRV o1 protein were detected in the same cells in this study, and localized to PRV inclusions. For orthoreoviruses like MRV, the viral replication process is also focused to cytoplasmic inclusions, called viral factories (15). The viral factories of orthoreoviruses have been shown to contain both large amounts of viral proteins and dsRNA. The globular inclusions detected in PRV infected RBC resemble the viral factories observed in MRV type 3 prototype strain Dearing (T3D) infected cells (Example 1). Thus, both content and morphology of PRV inclusions is similar to the viral factories of orthoreoviruses.

All salmonid erythrocytes are nucleated and retain ribosomes and a number of organelles while in circulation, thus competent to perform transcription and protein synthesis. However, during maturation and ageing there is an inverse relationship between loss of cytoplasmic organelles and increased hemoglobin content, accompanied by reduced aerobic metabolic rate. Both total RNA content and protein synthesis, under steady state conditions, are higher in young erythrocytes. Whether these changes alter their susceptibility to PRV infection is not known. The detection of large amounts of PRV protein in just a few cells during the *ex vivo* infection experiment could indicate that the susceptibility of RBC varies. Normally, a continuous cell line susceptible to a specific virus is used for viral propagation. However, these cell populations are homogenous and new cells are constantly produced by mitosis providing new permissive cells. In contrast, isolated primary erythrocytes from fish are non-dividing and more heterogeneous. Thus, a lower production of virus could be expected when using primary erythrocytes cultured *ex vivo.* The benefit of using primary RBC would be that it closely mimics the *in vivo* situation, and is well suited for the study of endogenous infection mechanisms.

In *ex vivo* infection experiments, PRV infected cells tended to have a rounder cellular and nuclear shape compared to uninfected erythrocytes. This was also noted in the *in vivo* infected RBC from i1 and i2 when kept in culture. Mature erythrocytes of Atlantic salmon have an ellipsoidal shape and a centrally located oval nucleus with compact chromatin. Immature erythrocytes, called polychromatocytes and reticulocytes, have a more oval cell shape, oval to rounded nucleus with less compact chromatin network indicating higher transcriptional activity (20). Whether the morphological difference between infected and uninfected cells observed in our experiments was due to different cell maturity prior to infection, or if it represents a morphological change induced by infection is not known. The naïve population (n1-n3) were described as RBC with more homogenous morphology compared to *in vivo* infected i1 and i2. In addition, weak staining of dsRNA and o1 appeared in RBC with ellipsoidal shape, but stronger stained cells tended to have a more circular morphology. This might suggest that the PRV infection itself induces a change in cellular and nuclear shape.

How PRV is transmitted from cell to cell is not known. Orthoreovirus release of progeny particles is thought to follow cell death and disruption of the plasma membrane and infections commonly results in cell lysis either by apoptotic or non-apoptotic cell death. Viral-induced apoptosis has been shown in several cell cultures and target tissues in vivo, including the heart. However, non-lytic persistent infections have also been demonstrated in cell cultures.

PRV have been associated with erythrocytic inclusion body syndrome (EIBS). EIBS is a collective term of several reports of viral inclusions in salmonid erythrocytes, and described in different salmonid species both in the freshwater and seawater phases from the Atlantic and Pacific coasts (11, 22, 23). The inclusions are observed in cytoplasm of infected erythrocytes and usually counts 1 to 3 and occasionally as many as 6 per cell. The most commonly used method in detection of EIBS is staining of blood smears with pinacyanol chloride, which binds nucleic acids, followed by detection of stained cytoplasmic inclusions using light microscopy. In the present study, similar inclusions, were observed using pinacyanol chloride staining, indication that previous diagnostic observations of EIBS may have detected PRV-infected erythrocytes.

Acridine orange, a nucleic acid selective fluorescent dye, have been used in the characterization of the inclusions observed in EIBS. Acridine orange emitts green fluorescence when bound to double strand (dsDNA or dsRNA) and red when bound to single stranded (ssDNA or ssRNA). It was reported that EIBS inclusions stained red, indicating the presence of single stranded nucleic acid RNA. In the present study dsRNA were demonstrated in the inclusions by using the monoclonal antibody against dsRNA. The reason for these different observations is unknown.

The development of EIBS has been studied in an experimental challenge of coho salmon (*O*. *kisuth*) monitoring development of erythrocytic inclusions and hematocrit values (11). The onset of EIBS, maximum number of inclusions, duration and recovery phase were shown to be temperature-related. The present study was conducted at 12 °C, and testing different temperatures could be used to further optimize the current ex *vivo* protocol.

Recent studies have shown that salmon erythrocytes can play an active role in the immune response and elicit antiviral responses to Poly(I:C) stimulation (58). We observe that PRV infection induces IFNα production and gene activation of Mx and RIG-I in ex *vivo* infected RBC, indicating mobilization of a robust antiviral response. The interferon (IFN) signaling mechanisms are well conserved in fish, and further lead to induction of Mx, which has been shown to inhibit viral replication and protect from grass carp reovirus infection . Whereas Mx has any inhibitory function towards PRV has not been subject to study. RIG-I upregulation is expected to sensitize the cells to cytosolic dsRNA, which may also limit further infection and replication in our cultures. This indicates that the antiviral response elicited by RBC may be a promising target for modulation, aiming to obtain a more efficient viral propagation in RBC *ex vivo.*
The ability to collect blood from the same individual fish throughout the course of infection experiments is ideal for the study of virus-host interactions. The *ex vivo* infection model described in this study could be further developed for more efficient viral propagation and will likely benefit future studies of PRV infection, replication and release from RBC. The use of primary RBC, known to be a major target cell for PRV *in vivo,* will produce information that closely reflects the *in vivo* situation.

### Example 4: Example of protocol for cultivation of PRV, in salmonid red blood cells (RBC)

### Preparation of inoculum

1. Collect blood sample in heparinized tube.
2. Centrifuge at 2000 g for 10 min at 4°C.
3. Remove plasma fraction.
4. Dilute blood 1:10 in PBS.
5. Either freeze/thaw or sonicate inoculum for cell lysis. (See below)

### Freeze thawing

1. Freeze / thaw three times at -80°C
2. Centrifuge 2000g/5min/4°C.
3. Collect the supernatant. Use as inoculum.

### Sonication

1. Sonicate on ice. (10X10 sec pulses at 25Hz, rest for 30 seconds between)
2. Centrifuge 2000g/5min/4°C.
3. Collect the supernatant. Use as inoculum.

### Cultivation

1. Plate 3mL naïve RBC in a 24 deep well plate using 10 mill / mL in L15 including 2% FCS and gentamicin.
2. Inoculate cells using 100 uL of the inoculum (freeze/thawed or sonicated)
3. Incubate for 7 or 14 days in InFors Ecotron shaker (200rpm) at 15°C.

### Sampling

1. Remove RBC suspension.
2. Centrifuge at 800g/5min/4°C.
3. Isolate RNA by any standard procedure for RNA isolation and ran RT-qPCR for analysis of PRV.

### REFERENCES

1. Kongtorp RT, Kjerstad A, Taksdal T, Guttvik A, Falk K. 2004. Heart and skeletal muscle inflammation in Atlantic salmon, Salmo salar L.: a new infectious disease. J.Fish.Dis. 27:351-358.
2. Kongtorp RT, Taksdal T, Lyngøy A. 2004. Pathology of heart and skeletal muscle inflammation (HSMI) in farmed Atlantic salmon Salmo salar. Dis.Aquat.Org. 59:217-224.
3. Palacios G, Lovoll M, Tengs T, Hornig M, Hutchison S, Hui J, Kongtorp RT, Savji N, Bussetti AV, Solovyov A, Kristoffersen AB, Celone C, Street C, Trifonov V, Hirschberg DL, Rabadan R, Egholm M, Rimstad E, Lipkin WI. 2010. Heart and Skeletal Muscle Inflammation of Farmed Salmon Is Associated with Infection with a Novel Reovirus. PloS one 5:e11487.
4. Markussen T, Dahle MK, Tengs T, Lovoll M, Finstad OW, Wiik-Nielsen CR, Grove S, Lauksund S, Robertsen B, Rimstad E. 2013. Sequence Analysis of the Genome of Piscine Orthoreovirus (PRV) Associated with Heart and Skeletal Muscle Inflammation (HSMI) in Atlantic Salmon (Salmo salar). PloS one 8:e70075.
5. Key T, Read J, Nibert ML, Duncan R. 2013. Piscine reovirus encodes a cytotoxic, non-fusogenic, integral membrane protein and previously unrecognized virion outer-capsid proteins. J.Gen.Virol. 94:1039-1050.
6. Garseth AH, Fritsvold C, Opheim M, Skjerve E, Biering E. 2013. Piscine reovirus (PRV) in wild Atlantic salmon, Salmo salar L., and sea-trout, Salmo trutta L., in Norway. J.Fish.Dis. 36:483-493.
7. Kibenge MJ, Iwamoto T, Wang Y, Morton A, Godoy MG, Kibenge FS. 2013. Whole-genome analysis of piscine reovirus (PRV) shows PRV represents a new genus in family Reoviridae and its genome segment S1 sequences group it into two separate sub-genotypes. Virol.J. 10:230.
8. Finstad ØW, Falk K, Løvoll M, Evensen Ø, Rimstad E. 2012. Immunohistochemical detection of piscine reovirus (PRV) in hearts of Atlantic salmon coincide with the course of heart and skeletal muscle inflammation (HSMI). Vet.Res. 43:27.
9. Mikalsen AB, Haugland O, Rode M, Solbakk IT, Evensen O. 2012. Atlantic salmon reovirus infection causes a CD8 T cell myocarditis in Atlantic salmon (Salmo salar L.). PLoS.One. 7:e37269.
10. Watanabe K, Karlsen M, Devoid M, Isdal E, Litlabo A, Nylund A. 2006. Virus-like particles associated with heart and skeletal muscle inflammation (HSMI). Dis.Aquat.Organ 70:183-192.
11. Piacentini SC, Rohovec JS, Fryer JL: Epizootiology of erythrocytic inclusion body syndrome. J Aquat Anim Health 1989, 1 :173-179.
12. Nibert ML, Duncan R: Bioinformatics of recent aqua- and orthoreovirus isolates from fish: Evolutionary gain or loss of FAST and fiber proteins and taxonomic implications PLoS One 2013, 8(7):e68607.
13. Attoui H, Fang Q, Mohd Jaafar F, Cantaloube JF, Biagini P, de Micco P, de Lamballerie X: Common evolutionary origin of aquareoviruses and orthoreoviruses revealed by genome characterization of Golden shiner reovirus, Grass carp reovirus, Striped bass reovirus and golden ide reovirus (genus Aquareovirus, family Reoviridae). J Gen Virol 2002, 83:1941-1951.
14. Bridle A, Nosworthy E, Polinski M, Nowak B. 2011. Evidence of an antimicrobial-immunomodulatory role of Atlantic salmon cathelicidins during infection with Yersinia ruckeri. PloS one 6:e23417.
15. Parker JS, Broering T J, Kim J, Higgins DE, Nibert ML. 2002. Reovirus core protein mu2 determines the filamentous morphology of viral inclusion bodies by interacting with and stabilizing microtubules. J.Virol. 76:4483-4496.
16. Dryden KA, Wang G, Yeager M, Nibert ML, Coombs KM, Furlong DB, Fields BN, Baker TS: Early steps in reovirus infection are associated with dramatic changes in supramolecular structure and protein conformation: analysis of virions and subviral particles by cryoelectron microscopy and image reconstruction. J Cell Biol 1993, 122:1023-1041.
17. Reiter OM, Frierson JM, Halvorson EE, Kobayashi T, Dermody TS, Stehle T. 2011. Crystal structure of reovirus attachment protein sigma1 in complex with sialylated oligosaccharides. PLoS.Pathog. 7:e1002166.
18. Garofalo F, Pellegrino D, Amelio D, Tota B: The Antarctic hemoglobinless icefish, fifty five years later: a unique cardiocirculatory interplay of disaptation and phenotypic plasticity. Comp Biochem Physiol A Mol Integr Physiol 2009, 154:10- 28.
19. Claver JA, Quaglia Al E. 2009. Comparative Morphology, Development, and Function of Blood Cells in Nonmammalian Vertebrates, p. 87-97, Journal of Exotic Pet Medicine Hematology, vol. 18. 50
20. Conroy DA: Basic atlas of Atlantic salmon (Sa/mo salar L.) blood cells. Carrickfergus: Patterson Peddie Consulting Ltd; 2006.
21. Lund SG, Phillips MC, Moyes CD, Tufts BL. 2000. The effects of cell ageing on protein synthesis in rainbow trout (Oncorhynchus mykiss) red blood cells. J.Exp.Biol. 203:2219-2228.
22. Michak P, Smith CE, Hopper K. 1992. Erythrocytic inclusion body syndrome: a light and electron microscopic study of infected erythrocytes of chinook Oncorhynchus tshawytscha and coho 0. kisutch salmon. Dis.Aquat.Organ 12:229-233.
23. Rodger HD. 2007. Erythrocytic inclusion body syndrome virus in wild Atlantic salmon, Salmo salar L. J.Fish Dis. 30:411-418.
24. WO2011041790.
25. K. Riji John et al. 2001. Characteristics of a new reovirus isolated from epizootic ulcerative syndrome infected snakehead fish. Dis Aquat Org Vol. 46: 83-92.

## Claims

1. An ex vivo method for propagating and isolating a piscine reovirus (PRV),
said method comprising ex vivo propagating and isolating said PRV in nucleated piscine erythrocytes.

2. An ex vivo method according to claim 1, wherein said nucleated piscine erythrocytes are Salmonidae erythrocytes.

3. An ex vivo method according to claim 2, wherein said Salmonidae is salmon or rainbow trout.

4. An ex vivo method according to any one of claims 1-3, said method comprising the steps of:
a) co-cultivation of nucleated piscine erythrocytes substantially free from reovirus with nucleated piscine erythrocytes infected with PRV and/or any other material containing a source of PRV to obtain a mixed culture;
b) incubating the mixed culture obtained in step a),
c) removing erythrocytes infected with PRV from said mixed culture of step b),
d) optionally repeating the procedure of steps a) to c), and
e) isolating PRV from the erythrocytes of step c).

5. An ex vivo method according to claim 4, wherein the incubation of step b) is performed for about 14 days or more, such as about 4-45 days.

6. An ex vivo method of preparing a vaccine composition said method comprising the method of any one of claims 1-5 followed by inactivating the isolated PRV obtained in step e) and thereafter preparing a vaccine composition containing said inactivated PRV.

7. An ex vivo method according to claim 6, wherein said vaccine composition is to be used for the treatment and/or prevention of Heart and Skeletal Muscle Inflammation (HSMI).

8. An ex vivo method for diagnosing PRV infection or the presence of PRV virus in fish of a species of Salmonidae, such as salmon, said method comprising:
a) isolating erythrocytes from a biological blood sample from said fish of a species of Salmonidae,
b) detecting the presence of piscine reovirus (PRV) in said erythrocytes obtained from step a).

9. An ex vivo method according to claim 8, wherein said detection is performed by RT-qPCR.

10. Use *ex vivo* of nucleated piscine erythrocytes, such as Salmonidae erythrocytes, more particular salmon or rainbow trout erythrocytes for propagating piscine reovirus (PRV).

## Patentansprüche

1. Ex-vivo-Verfahren zur Vermehrung und Isolierung eines Piscine Reovirus (PRV), wobei das Verfahren Vermehren und Isolieren des PRV ex vivo in nukleierten Piscine-Erythrozyten umfasst.

2. Ex-vivo-Verfahren gemäß Anspruch 1, wobei die nukleierten Piscine-Erythrozyten Salmonidae-Erythrozyten sind.

3. Ex-vivo-Verfahren gemäß Anspruch 2, wobei die Salmonidae Lachs- oder Regenbogenforelle ist.

4. Ex-vivo-Verfahren gemäß einem der Ansprüche 1 bis 3, wobei das Verfahren die folgenden Schritte umfasst:
a) Co-Kultivierung von nukleierten Piscine-Erythrozyten, die im Wesentlichen frei von Reovirus sind, mit nukleierten Piscine-Erythrozyten, infiziert mit PRV, und/oder einem beliebigen anderen Material, das eine PRV-Quelle enthält, unter Erhalt einer Mischkultur;
b) Inkubieren der in Schritt a) erhaltenen Mischkultur,
c) Entfernen von Erythrozyten, die mit PRV infiziert sind, aus der Mischkultur von Schritt b),
d) gegebenenfalls Wiederholen der Arbeitsgänge der Schritte a) bis c) und
e) Isolieren von PRV aus den Erythrozyten von Schritt c) .

5. Ex-vivo-Verfahren gemäß Anspruch 4, wobei die Inkubation von Schritt b) für etwa 14 Tage oder mehr, zum Beispiel etwa 4-45 Tage, durchgeführt wird.

6. Ex-vivo-Verfahren zur Herstellung einer Vakzin-Zusammensetzung, wobei das Verfahren das Verfahren gemäß einem der Ansprüche 1 bis 5, gefolgt von Inaktivieren des isolierten PRV, das in Schritt e) erhalten wurde, und danach Herstellen einer Vakzin-Zusammensetzung, die das inaktivierte PRV enthält, umfasst.

7. Ex-vivo-Verfahren gemäß Anspruch 6, wobei die Vakzin-Zusammensetzung für die Behandlung und/oder Prävention von Herz- und Skelettmuskelentzündung (Heart and Skeletal Muscle Inflammation (HSMI)) zu verwenden ist.

8. Ex-vivo-Verfahren zum Diagnostizieren einer PRV-Infektion oder des Vorliegens von PRV-Virus in Fisch einer Spezies von Salmonidae, zum Beispiel Lachs, wobei das Verfahren umfasst:
a) Isolieren von Erythrozyten aus einer biologischen Blutprobe von dem Fisch einer Spezies von Salmonidae,
b) Detektieren des Vorliegens von Piscine Reovirus (PRV) in den Erythrozyten, die aus Schritt a) erhalten wurden.

9. Ex-vivo-Verfahren gemäß Anspruch 8, wobei die Detektion durch RT-qPCR durchgeführt wird.

10. Ex-vivo-Verwendung von nukleierten Piscine-Erythrozyten, zum Beispiel Salmonidae-Erythrozyten, spezieller Lachs- oder Regenbogenforellen-Erythrozyten, zur Vermehrung von Piscine-Reovirus (PRV).

## Revendications

1. Procédé *ex vivo* pour la propagation et l'isolement d'un réovirus de poisson (PRV), ledit procédé comprenant la propagation *ex vivo* et l'isolement dudit PRV dans des érythrocytes nucléés de poisson.

2. Procédé *ex vivo* selon la revendication 1, dans lequel les érythrocytes nucléés de poisson sont des érythrocytes de Salmonidés.

3. Procédé *ex vivo* selon la revendication 2, dans lequel lesdits Salmonidés sont le saumon ou la truite arc-en-ciel.

4. Procédé *ex vivo* selon l'une quelconque des revendications 1 à 3, ledit procédé comprenant les étapes suivantes :
a) la coculture des érythrocytes nucléés de poisson sensiblement dépourvus de réovirus avec des érythrocytes nucléés de poisson infectés avec le PRV et/ou tout autre matériau contenant une source de PRV pour obtenir une culture mixte ;
b) l'incubation de la culture mixte obtenue dans l'étape a),
c) l'élimination des érythrocytes infectés avec le PRV à partir de ladite culture mixte de l'étape b),
d) la répétition éventuelle de la procédure des étapes a) à c), et
e) l'isolement du PRV à partir des érythrocytes de l'étape c).

5. Procédé *ex vivo* selon la revendication 4, dans lequel l'incubation de l'étape b) est effectuée pendant environ 14 jours ou plus, comme environ 4 à 45 jours.

6. Procédé *ex vivo* de préparation d'une composition vaccinale, ledit procédé comprenant le procédé selon l'une quelconque des revendications 1 à 5 suivi de l'inactivation du PRV isolé obtenu dans l'étape e) et ensuite la préparation d'une composition vaccinale contenant ledit PRV inactivé.

7. Procédé *ex vivo* selon la revendication 6, dans lequel ladite composition vaccinale doit être utilisée pour le traitement et/ou la prévention de l'inflammation des muscles squelettiques et cardiaques (IMSC).

8. Procédé *ex vivo* pour le diagnostic d'une infection à PRV ou de la présence du virus PRV dans un poisson d'une espèce de Salmonidés, comme le saumon, ledit procédé comprenant :
a) l'isolement des érythrocytes à partir d'un échantillon de sang biologique provenant dudit poisson d'une espèce de Salmonidés,
b) la détection de la présence de réovirus de poisson (PRV) dans lesdits érythrocytes obtenus à partir de l'étape a).

9. Procédé *ex vivo* selon la revendication 8, dans lequel ladite détection est effectuée par RT-qPCR.

10. Utilisation *ex vivo* d'érythrocytes nucléés de poisson, comme des érythrocytes de Salmonidés, plus particulièrement des érythrocytes de saumon ou de truite arc-en-ciel pour la propagation du réovirus de poisson (PRV).
